# EUROPEAN PATENT APPLICATION

(11) **EP 4 821 505 A1**
(43) Date of publication of application: **14.10.2026**
(21) Application number: 24901000.0
(22) Date of filing: 02.12.2024
(51) Int. Cl.: A61K 39/04, A61K 38/16, A61P 31/06, C07K 14/35

(54) **TUBERCULOSIS VACCINE COMPOSITION COMPRISING FUSION PROTEIN INDUCING BACTERICIDAL IMMUNITY AGAINST MYCOBACTERIUM TUBERCULOSIS**

(30) Priority: 06.12.2023 KR 20230175586
(71) Applicant: Myco-Rapha Inc., Daejeon 35015 (KR)
(72) Inventor: KIM, Hwa Jung, Daejeon 34961 (KR); CHOI, Seung A, Daejeon 34090 (KR); CHOI, Han Gyu, Daejeon 35359 (KR); BAEK, Yong Woo, Gimcheon-si, Gyeongsangbuk-do 39652 (KR)
(74) Representative: Kraus & Lederer PartGmbB
(86) International application number: PCT/KR2024/019479
(87) International publication number: WO 2025/121814

(57) **Abstract**

The present invention relates to a tuberculosis vaccine composition comprising a fusion protein inducing bactericidal immunity against Mycobacterium tuberculosis. Specifically, a tuberculosis vaccine composition comprising a fusion protein of Rv2299cD2D3-ESAT6-Ag85B(REA) or Rv2299cD2D3-Rv3463-Ag85B(RRA) induces sterilization of 99% or more of Mycobacterium tuberculosis, and thus can prevent adult tuberculosis and can be used as a vaccine for infants/toddlers.

## Description

### Technical Field

The present disclosure relates to a tuberculosis vaccine composition including a fusion protein inducing sterilizing immunity against *Mycobacterium tuberculosis*.

### Background Art

Tuberculosis, which is an infectious disease caused by *Mycobacterium tuberculosis* (MTB), is an infectious disease that has claimed the greatest number of human lives to date. Even recently, 1.5 million people worldwide have died from tuberculosis, and in terms of tuberculosis incidence and mortality, Korea is one of the countries with a serious tuberculosis problem among the 32 OECD member countries.

The most effective strategy for solving this tuberculosis problem is development of a safe and highly effective vaccine. However, the preventive effect of *Mycobacterium bovis* Bacillus Calmette-Guérin (BCG), which is the only vaccine against tuberculosis, has been controversial, ranging from 0 to 80% depending on the reporter, and it has been reported that BCG has no preventive effect against reactivation of latent tuberculosis or adult tuberculosis. However, because it has a preventive effect against severe tuberculosis in children, it is administered in many countries, but a vaccine having efficacy far superior to that of BCG has not yet been developed. In addition, the goal of the World Health Organization (WHO) End TB strategy is to reduce the tuberculosis incidence rate by 90% and the mortality rate by 95% by 2035 based on 2015. Korea is also promoting a Tuberculosis Elimination 2030 plan with a target of less than 10 tuberculosis cases per 100,000 persons. In order to create a world free of tuberculosis as described above, development of a tuberculosis vaccine that may serve as a game changer with excellent preventive efficacy is essential.

Currently, about 10 types of tuberculosis vaccines are undergoing clinical trials worldwide, and when these are classified according to function, they include: ① live attenuated vaccines for replacing BCG, which is a prime vaccine; ② BCG booster vaccines applying an immunopotentiator or a viral vector; and ③ immunotherapeutic vaccines for shortening the treatment period or suppressing recurrence. Development of live attenuated vaccines for replacing BCG is mainly in the form of genetically recombinant BCG strains and *Mycobacterium tuberculosis* strains in which two genes are simultaneously deleted. These live attenuated vaccines contain many antigens as compared with subunit vaccines, and thus may be expected to have a high protective effect because they induce diverse immune responses. However, development of recombinant strains or gene-deficient strains takes a long time due to issues such as removal of antibiotic resistance markers and proof of safety, and even after development, strict quality control is required in the process of manufacturing vaccine strains. Due to these limitations, development of tuberculosis vaccines has been focused on development of protein-based subunit vaccines, and such subunit vaccines have limitations in replacing BCG and are therefore being developed mainly as BCG booster vaccines.

Accordingly, in order to become a more effective vaccine, when a vaccinated animal or person is exposed to *Mycobacterium tuberculosis,* sterilizing immunity, which is an immune response that completely eliminates the invading bacteria, should be induced, and in such a case, a protective efficacy of 95% or more may be expected. However, among the vaccines currently undergoing clinical trials, there is still no vaccine capable of inducing such sterilizing immunity.

Antigens applied to the development of conventional subunit vaccines currently undergoing clinical trials are mainly T cell-stimulating antigens such as Ag85 or ESAT6 antigens, PE/PPE antigens, some virulence-related antigens, and protein antigens related to induction of latent tuberculosis, and the H56 and ID93 vaccines are multicomponent protein vaccines in which proteins mainly activating T cells or proteins related to bacterial virulence are fused with proteins related to induction of latent tuberculosis. However, considering the fact that *Mycobacterium tuberculosis* cannot be completely eliminated even in immunocompetent individuals, vaccines using only T cells have limitations in inducing sterilizing immunity, and thus research thereon is urgently needed.

### Disclosure of Invention

### Technical Problem

The present disclosure is directed to providing a tuberculosis vaccine composition including a fusion protein inducing sterilizing immunity against *Mycobacterium tuberculosis*.

### Solution to Problem

In order to achieve the goal, the present disclosure provides a tuberculosis vaccine composition including an Rv2299cD2D3-ESAT6-Ag85B (REA) fusion protein. The Rv2299cD2D3-ESAT6-Ag85B (REA) fusion protein is characterized in that it is encoded by the nucleotide sequence of SEQ ID NO: 1. In addition, the Rv2299cD2D3-ESAT6-Ag85B (REA) fusion protein is characterized in that it includes the amino acid sequence of SEQ ID NO: 2.

The present disclosure also provides a tuberculosis vaccine composition including an Rv2299cD2D3-Rv3463-Ag85B (RRA) fusion protein. The Rv2299cD2D3-Rv3463-Ag85B (RRA) fusion protein is characterized in that it is encoded by the nucleotide sequence of SEQ ID NO: 3. The Rv2299cD2D3-Rv3463-Ag85B (RRA) fusion protein is characterized in that it includes the amino acid sequence of SEQ ID NO: 4.

The tuberculosis vaccine composition according to the present disclosure induces sterilization of 99% or more against *Mycobacterium tuberculosis*.

The tuberculosis vaccine composition according to the present disclosure may induce sterilizing immunity against *Mycobacterium tuberculosis* by increasing the proportion of antigen-specific CD4⁺ T cells producing IFN-γ⁺IL-2⁺TNF-α⁺ or IL-2⁺TNF-α⁺.

The present disclosure provides a recombinant polynucleotide for sterilizing immunity against tuberculosis, the recombinant polynucleotide including the nucleotide sequence set forth in SEQ ID NO: 1 or 3. The present disclosure also provides a recombinant vector carrying the recombinant polynucleotide. The vector may be composed of pET-22b(+)_Rv2299cD2D3-ESAT6-Ag85B or pET-22b(+)_Rv2299cD2D3-Rv3463-Ag85B, and may be constructed using a commonly available plasmid, phage, or the like, including the sequence comprising the antigens of Rv2299cD2D3, ESAT6, and Ag85B, or Rv2299cD2D3, Rv3463, and Ag85B. The vector may transform a commonly available host cell including *E. coli*, and in particular, the Rv2299cD2D3-ESAT6-Ag85B (REA) fusion protein or the Rv2299cD2D3-Rv3463-Ag85B (RRA) fusion protein may be obtained from a host cell specialized for protein production and secretion.

The present disclosure provides a recombinant polypeptide for sterilizing immunity against tuberculosis, the recombinant polypeptide including the amino acid sequence set forth in SEQ ID NO: 2 or 4. The recombinant polypeptide for sterilizing immunity against tuberculosis comprising the amino acid sequence set forth in SEQ ID NO: 2 or 4 may be the Rv2299cD2D3-ESAT6-Ag85B (REA) fusion protein or the Rv2299cD2D3-Rv3463-Ag85B (RRA) fusion protein.

The tuberculosis vaccine composition comprising a fusion protein inducing sterilizing immunity against *Mycobacterium tuberculosis* according to the present disclosure may replace BCG, and accordingly may be used not only for prevention of adult tuberculosis but also as a vaccine for infants and children.

### Advantageous Effects of Invention

The present disclosure relates to a tuberculosis vaccine composition comprising a fusion protein inducing sterilizing immunity against *Mycobacterium tuberculosis*, and more specifically, through a tuberculosis vaccine composition comprising the fusion protein of Rv2299cD2D3-ESAT6-Ag85B (REA) or Rv2299cD2D3-Rv3463-Ag85B (RRA), sterilization of 99% or more against *Mycobacterium tuberculosis* is induced, and thus the composition may be used not only for prevention of adult tuberculosis but also as a vaccine for infants and children.

### Brief Description of Drawings

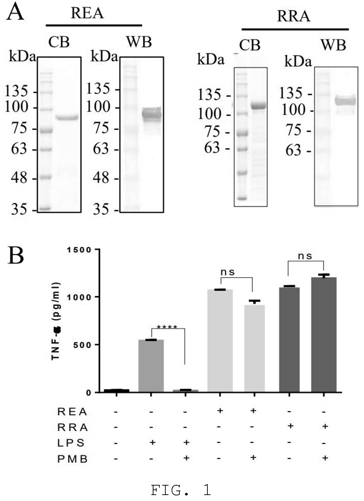

FIG. 1 shows graphs confirming purification and endotoxin of REA and RRA. (A) Results of Coomassie blue (CB) staining and Western blot (WB) analysis with an anti-His antibody after SDS-PAGE of *E. coli* extracts transformed with pET plasmids containing REA and RRA. (B) Comparison of TNF-α production by BMDMs treated with LPS, RRA, or REA depending on whether polymyxin B pretreatment was performed (****p < 0.0001).

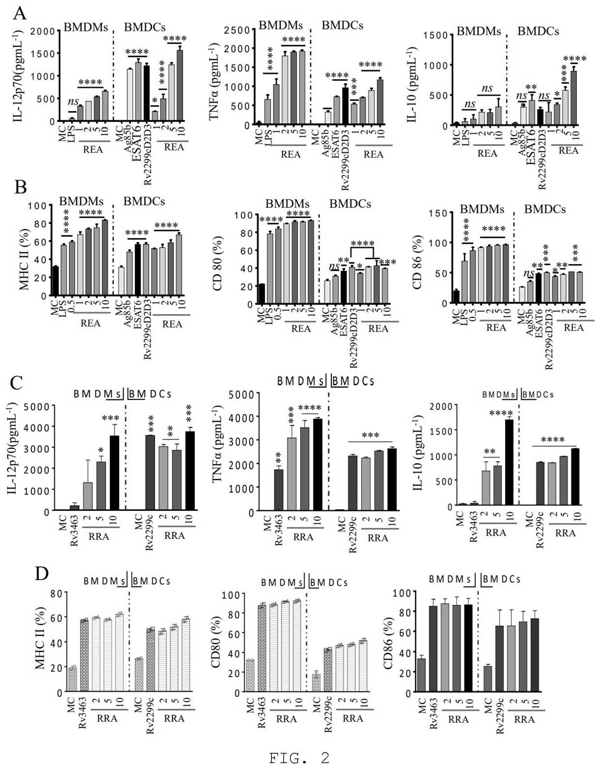

FIG. 2 shows graphs illustrating the effects of REA and RRA on activation of antigen-presenting cells. (A) Changes in IL-12p70, TNFα, and IL-10 production when BMDMs/BMDCs were stimulated with LPS, Ag85b, ESAT6, Rv2299cD2D3, and REA at varying concentrations. (B) Expression of surface markers MHC II, CD80, and CD86 when BMDMs/BMDCs were stimulated with LPS, Ag85b, ESAT6, Rv2299cD2D3, and REA at varying concentrations (bar graphs are expressed as percentages for each surface molecule of Anti-Mo F4/80 BMDM/CD11c+ BMDC cells). (C) Changes in IL-12p70, TNFα, and IL-10 production when BMDMs/BMDCs were stimulated with Rv3463, Rv2299c, and RRA at varying concentrations. (D) Expression of surface markers MHC II, CD80, and CD86 when BMDMs/BMDCs were stimulated with Rv3463, Rv2299c, and RRA at varying concentrations. (The data shown are means ± SD (n = 3); *p < 0.05, **p < 0.01, or ***p < 0.001, as compared with the medium control (MC)).

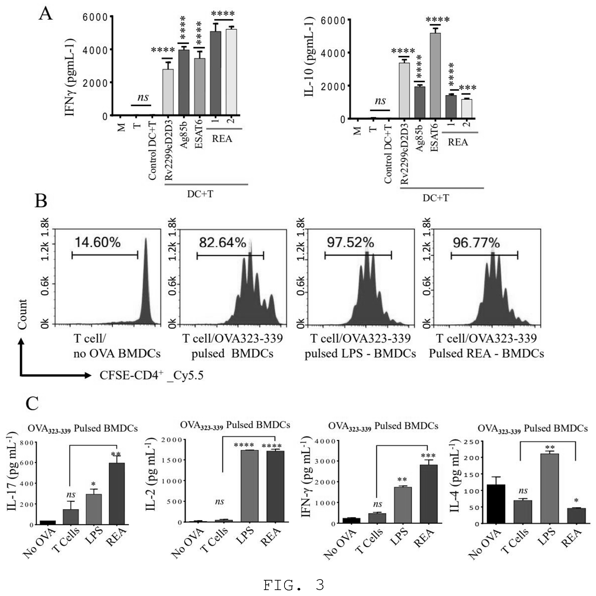

FIG. 3 shows graphs illustrating the Th1 response induced by REA-matured DCs. (A) Changes in IFNγ and IL-10 production after co-culturing unstimulated DCs (control DCs) and DCs stimulated with Rv2299cD2D3 (5 µg/mL), Ag85B (5 µg/mL), ESAT6 (2 µg/mL), or REA (1 or 2 µg/mL) with naïve T cells for 72 hours (DC:T cell = 1:10). (B) Proliferation of T cells pulsed with OVA323-339 (1 µg/mL) after co-culturing CFSE-labeled transgenic OVA-specific CD4⁺ T cells isolated from B6.Cg-Tg(TcraTcrb)425Cbn/J mice with DCs treated with REA (2 µg) or LPS (100 ng/mL) for 96 hours. (Controls are T cells alone or T cells co-cultured with untreated DCs). (C) Quantitative comparison of IFNγ, IL-2, IL-17, and IL-4 in the culture supernatants under each condition by ELISA. (The data shown are means ± SD (n = 3); *p < 0.05, **p < 0.01, ***p < 0.001, or ****p < 0.0001, as compared with the appropriate control (T cells/OVA257-264-pulsed DCs or T cells/OVA323-339-pulsed DCs), ns.: no significant difference).

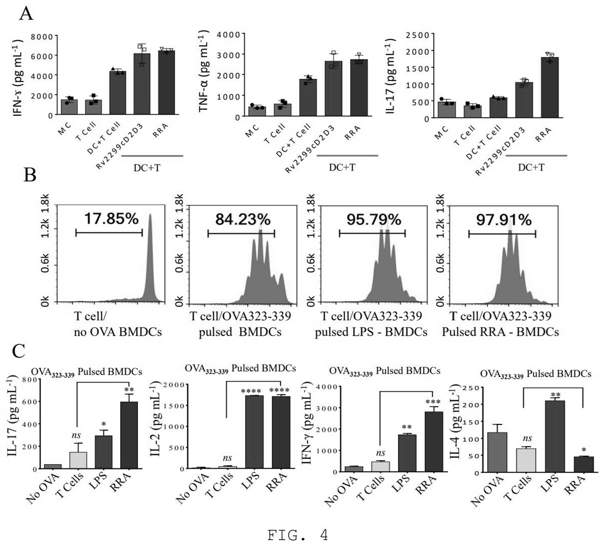

FIG. 4 shows graphs illustrating the Th1 response induced by RRA-matured DCs. (A) Changes in IFNγ, TNFα, and IL-17 production after co-culturing unstimulated DCs (control DCs) and DCs stimulated with Rv2299cD2D3 (5 µg/mL) or RRA (2 µg/mL) with naïve T cells for 72 hours (DC:T cell = 1:10). (B) Proliferation of T cells pulsed with OVA323-339 (1 µg/mL) after co-culturing CFSE-labeled transgenic OVA-specific CD4⁺ T cells isolated from B6.Cg-Tg(TcraTcrb)425Cbn/J mice with DCs treated with RRA (2 µg) or LPS (100 ng/mL) for 96 hours. (Controls are T cells alone or T cells co-cultured with untreated DCs). (C) Quantitative comparison of IL-17, IL-2, IFNγ, and IL-4 in the culture supernatants under each condition by ELISA. (The data shown are means ± SD; *p < 0.05, **p < 0.01, ***p < 0.001, or ****p < 0.0001, as compared with the appropriate control (T cells/OVA257-264-pulsed DCs or T cells/OVA323-339-pulsed DCs), ns.: no significant difference).

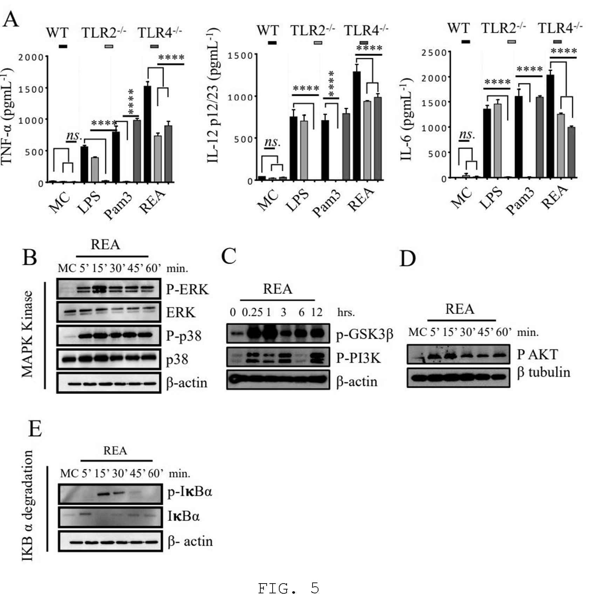

FIG. 5 shows results demonstrating activation of BMDMs by REA through Toll-like receptors 2 and 4 (TLR2/TLR4). (A) Comparison of TNF-α, IL-12, and IL-6 production in culture supernatants by ELISA after treating BMDMs derived from WT, TLR2-/-, and TLR4-/- mice with REA (5 µg/mL), LPS (100 ng/mL), or the TLR2 agonist Pam3CSK4 (100 ng/mL) for 24 hours. (One-way ANOVA with Tukey's multiple comparison test was used, and the indicated p values compare cytokines released by BMDMs from TLR2-/- and TLR4-/- mice with those released by WT-derived BMDMs in the presence or absence of antigen). (B, C) Immunoblotting results of membrane fractions of BMDM cells (1×10⁵/well) treated with REA (5 µg/mL) for the indicated times for (B) phospho-p38 (p-p38), p38, phospho-ERK1/2 (p-ERK1/2), and ERK1/2, and (C) GSK3β, P-PI3K, (D) P-AKT, and (E) p-IκBα and IκBα.

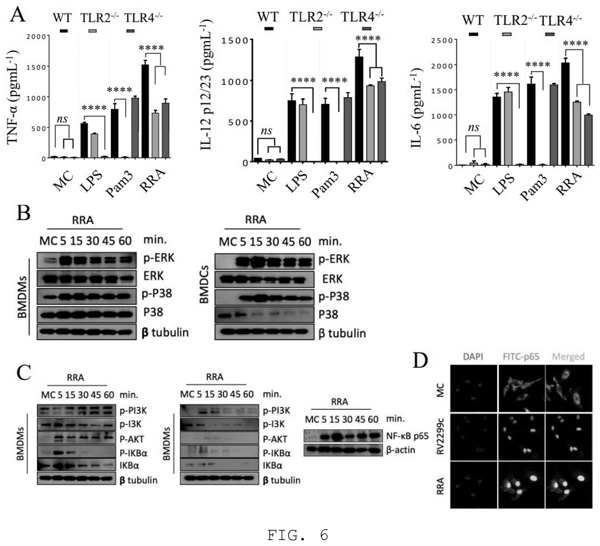

FIG. 6 shows results demonstrating activation of BMDMs by REA through Toll-like receptors 2 and 4 (TLR2/TLR4). (A) Comparison of TNF-α, IL-12, and IL-6 production in culture supernatants by ELISA after treating BMDMs derived from WT, TLR2-/-, and TLR4-/- mice with RRA (5 µg/mL), LPS (100 ng/mL), or the TLR2 agonist Pam3CSK4 (100 ng/mL) for 24 hours (one-way ANOVA with Tukey's multiple comparison test; the indicated p values compare cytokines released by BMDMs from TLR2-/- and TLR4-/- mice with those released by WT-derived BMDMs in the presence or absence of antigen, ns: no significant difference). (B, C) Immunoblotting results of membrane fractions of BMDM and BMDC cells (1×10⁵/well) treated with RRA (5 µg/mL) for the indicated times for (B) phospho-p38 (p-p38), p38, phospho-ERK1/2 (p-ERK1/2), and ERK1/2, and (C) p-PI3K, P-PI3K, p-AKT, p-IκBα, IκBα, and NFκB p65. (D) Intracellular localization of the p65 subunit of NF-κB detected by immunofluorescence.

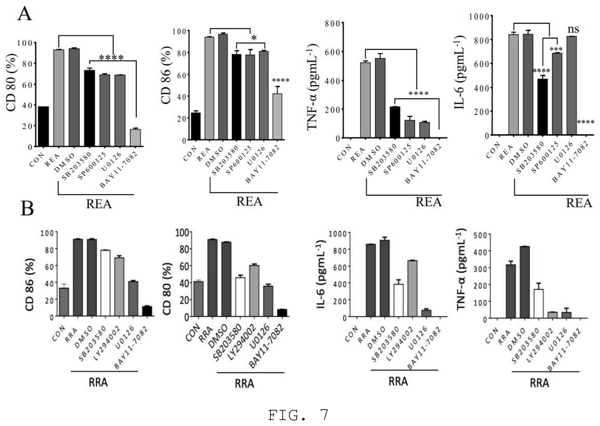

FIG. 7 shows the effects of pharmacological inhibitors on surface marker expression and cytokine production of BMDMs treated with REA and RRA. (A, B) BMDMs (1×10⁶/well) were treated for 1 hour with pharmacological inhibitors of p38 (SB203580, 20 µM), ERK1/2 (U0126, 10 µM), and NF-κB (Bay11-7082, 5 µM), or DMSO (vehicle control), and then treated with REA (A) or RRA (B) (5 µg/mL) for 24 hours, followed by measurement of levels of co-stimulatory surface markers (CD80, CD86) by flow cytometry and TNF-α and IL-6 in culture supernatants by ELISA. (The data shown are means ± SD (n = 3), *p < 0.05, **p < 0.01, or ***p < 0.001).

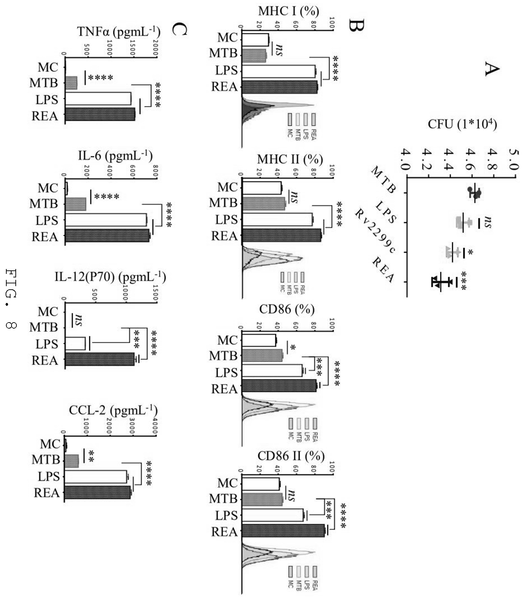

FIG. 8 shows the effects of REA on inhibition of intracellular growth of MTB in BMDMs and activation of infected cells. (A) Intracellular MTB growth under LPS, Rv2299c, and REA conditions. (B) Analysis of antigen presentation ability during infection (in BMDMs infected with H37Rv [MOI:1]) with respect to expression of surface molecules by two-color flow cytometry. (C) ELISA results of cytokine production in the culture supernatants shown in (B). (n = 3, shown as mean ± SD, analyzed using one-way ANOVA and Tukey's post hoc test, p values compare surface molecules and cytokine levels between MC and antigen-treated BMDMs, *p < 0.05, **p < 0.01, ***p < 0.001, ****p < 0.0001).

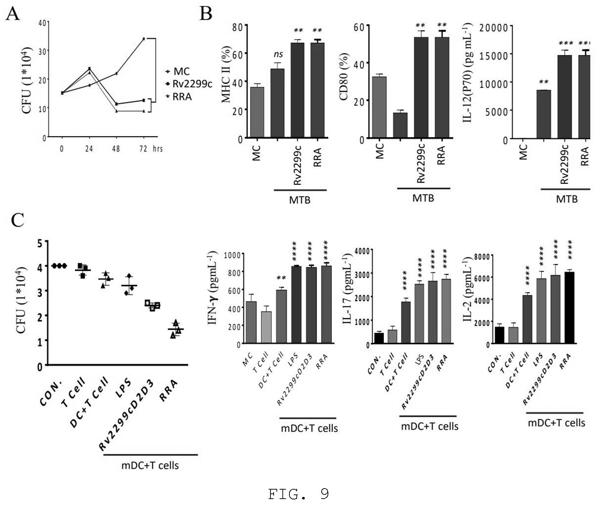

FIG. 9 shows the effect of T cells matured by RRA on inhibition of intracellular bacterial growth. (A) Intracellular MTB growth after culturing H37Rv-infected BMDMs with LPS, Rv2299c, or RRA. (B) Analysis of expression of surface molecules MHC II, CD80, and IL-12(P70) by two-color flow cytometry to assess antigen presentation ability during infection (in BMDMs infected with H37Rv [MOI:1]). (C) Intracellular MTB growth and ELISA results of cytokine production in the culture supernatants after co-culturing MTB-infected BMDMs with T cells activated by BMDCs matured with LPS, Rv2299cD2D3, or RRA. (n = 3, shown as mean ± SD, analyzed using one-way ANOVA and Tukey's post hoc test, p values compare surface molecules and cytokine levels between MC and antigen-treated BMDMs, *p < 0.05, **p < 0.01, ***p < 0.001, ****p < 0.0001).

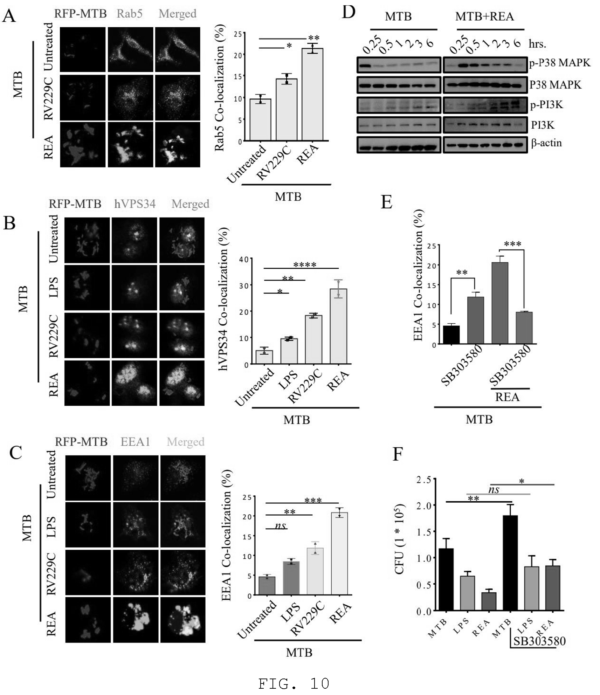

FIG. 10 shows the effect of REA on early endosome maturation in MTB-infected BMDMs. (A) Confocal microscopy imaging of endosomal colocalization after staining RFP-MTB ([MOI:1]) H37Rv-infected BMDMs treated with LPS, Rv2299c, or REA with Ras-related protein 5 (Rab5), with the bar graph showing the ratio of Rab5 colocalization. (B, C) Under the same settings as in (A), cells were stained with anti-phosphoinositide 3-kinase vacuolar protein sorting 34 (hVPS34) (B) or anti-EEA1 (C). (D) Western blot results showing time-dependent phosphorylation and total protein levels of Class I PI3K and p38 MAPK signaling components. (E) Degree of colocalization of anti-EEA1 after pretreating BMDMs with SB303580 followed by REA treatment during H37Rv infection. Phagosomes containing MTB were observed by confocal microscopy for colocalization with the indicated markers. (F) Growth of intracellular bacteria in MTB-infected BMDMs treated with REA or LPS after SB303580 pretreatment. (*p < 0.05 and **p < 0.01).

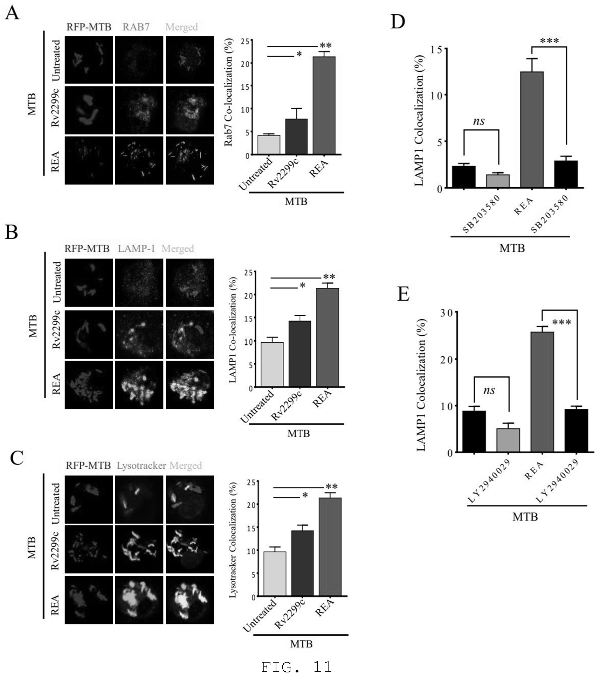

FIG. 11 shows the effect of REA on phagosomal acidification. (A, B, C) BMDMs (1×10⁶/well) infected with RFP-MTB [MOI:1] were treated with Rv2299c or REA (5 µg mL⁻¹), followed by staining of phagosomes containing RAB7 (A), LAMP1 (B), Lyso Tracker^{™} Green DND-26 (C), and MTB. (D, E) BMDMs were infected with RFP-H37Rv [MOI:1] for 4 hours and treated 1 hour before Rv2299c or REA treatment with SB203580 p38 inhibitor (D) or LY2940029 PI3K inhibitor (E), followed by determination of the ratio of LAMP colocalization. (*p < 0.05, **p < 0.01, and ***p < 0.001, comparing Rv2299c- or REA-treated infected BMDMs with untreated MTB H37Rv-infected BMDMs).

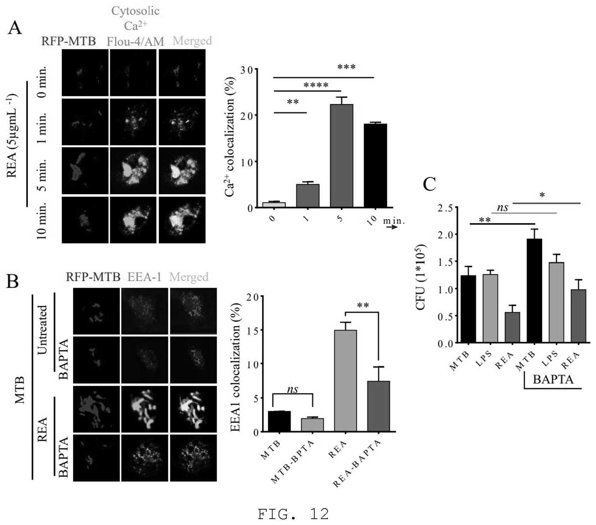

FIG. 12 shows the effect of REA on the increase in intracellular Ca²⁺ that induces EEA1 colocalization in MTB-infected BMDMs. (A) Colocalization of calcium after REA treatment for the indicated periods following Fluo-4/AM loading of BMDMs infected with RFP-MTB [MOI:1]. (**p < 0.01, ***p < 0.01, and ****p < 0.001, p values represent time-dependent intensity of Fluo-4/AM). (B) Colocalization of EEA1 after treating cells with REA following application of BAPTA/AM, a Ca²⁺ chelator, in (A). (C) Intracellular MTB growth according to BAPTA/MA treatment. (*p < 0.05, **p < 0.01, ns: no statistically significant difference).

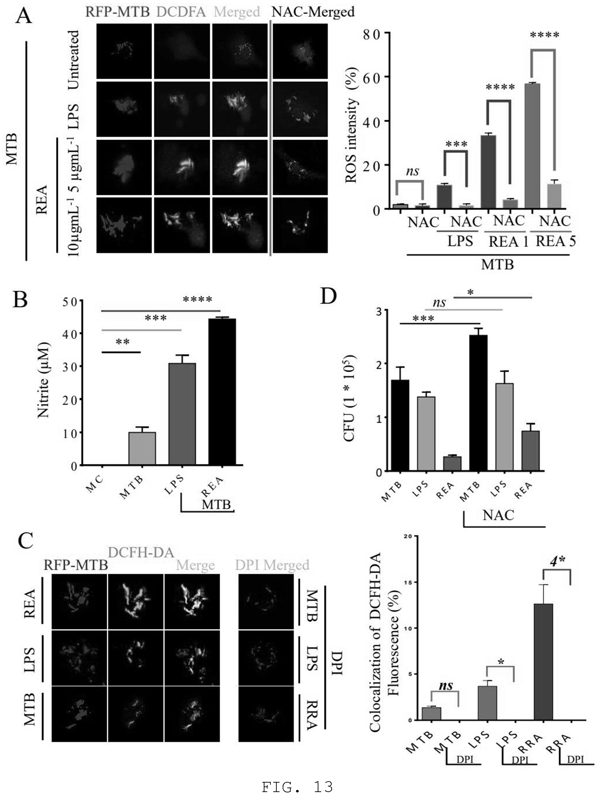

FIG. 13 shows the effect of REA on intracellular ROS and NO production. (A) Intracellular ROS levels in BMDMs infected with RFP-MTB [MOI:1] were measured by DCDF-based staining according to NAC treatment. The bar graph shows DCF fluorescence intensity calculated using ImagJ.exe. (B) NO levels in culture supernatants were measured after treatment with REA or LPS for 72 hours regardless of infection. (C) After DPI pretreatment, BMDMs (1×10⁶/well) infected with RFP-MTB (MOI:1) were stimulated with LPS or REA, and intracellular ROS levels were measured by DCDF-based staining. (D) Intracellular bacterial growth was measured according to NAC treatment 1 hour before infection. (*p < 0.05, **p < 0.01, ***p < 0.001, and ****p < 0.0001, ns: no statistically significant difference).

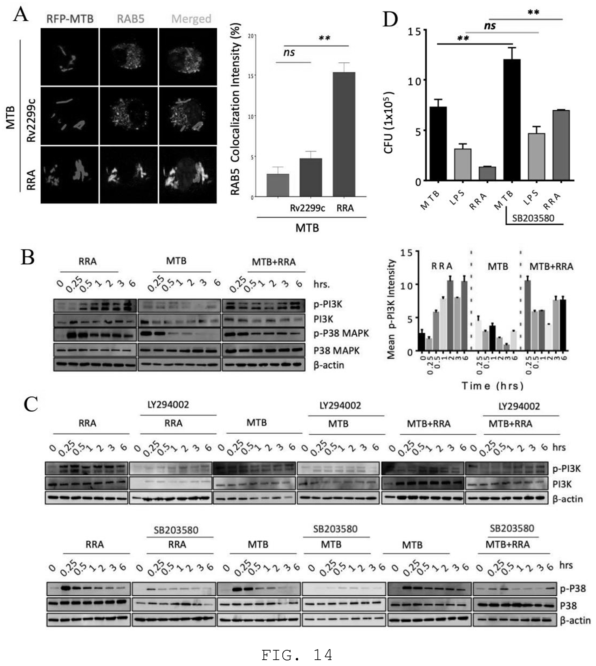

FIG. 14 shows the effects of RRA on RAB5 and on activation of PI3K and p38 MAPK. (A) Confocal microscopy imaging of endosomal colocalization after staining RAB5 in BMDM cells infected with RFP-MTB H37Rv ([MOI:1]) and treated with RRA or Rv2299c. The histogram shows the ratio of RAB5 colocalization induced by REA relative to Rv2299c and untreated controls. (B) Western blot results showing time-dependent phosphorylation and total protein levels of Class I PI3K and p38 MAPK signaling components. (C) Western blot results showing time-dependent phosphorylation of Class I PI3K according to SB303580 or LY2940029 treatment. (D) Growth of bacteria within MTB-infected BMDM cells treated with RRA or LPS according to SB303580 treatment. (**p < 0.01, ns: no statistically significant difference).

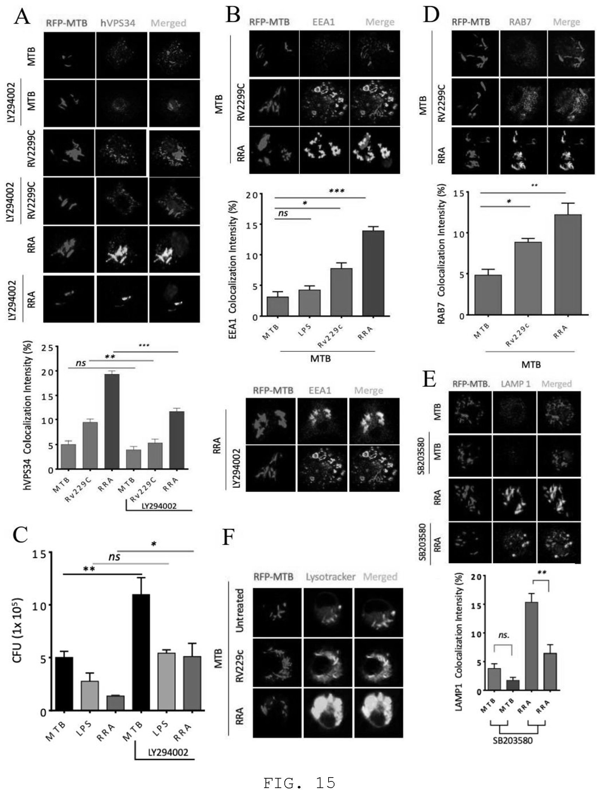

FIG. 15 shows that RRA recruits hVPS34, EEA1, and RAB7 through activation of PI3K and induces phagolysosomal fusion. (A, B, D, E, F) BMDM cells infected with RFP-MTB H37Rv ([MOI:1]) were treated with RRA or Rv2299c together with LY2940029 or SB203580, followed by staining with anti-hVPS34 (A), anti-EEA1 (B), anti-Rab7 (D), anti-LAMP1 (E), or Lyso Tracker^{™} Green DND-26 (F), and endosomal colocalization was imaged by confocal microscopy. The bar graphs show the ratio of colocalization of each detected protein. (C) Growth of intracellular *Mycobacterium tuberculosis* according to LY2940029 treatment. (*p < 0.05, **p < 0.01, ***p < 0.001, ns: no statistically significant difference).

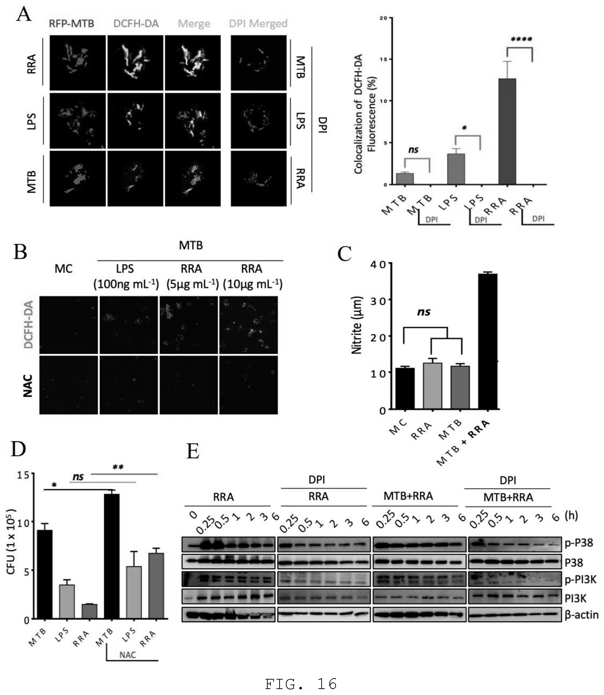

FIG. 16 shows the effect of RRA on intracellular ROS and NO production. (A, B) After stimulating BMDMs (1×10⁶/well) infected with RFP-MTB [MOI:1] with LPS or RRA, intracellular ROS levels were measured by DCDF fluorescence-based staining according to DPI (A) or NAC (B) pretreatment, and the bar graphs show DCF fluorescence intensity calculated using ImagJ.exe. (C) NO levels in culture supernatants at 72 hours after infection and RRA treatment. (D) Measurement of intracellular *Mycobacterium tuberculosis* in BMDMs treated with RRA or LPS according to NAC pretreatment before infection. (E) Immunoblot analysis of phosphorylation of p38 and PI3K in MTB-infected and RRA-treated cells according to DPI pretreatment. (*p < 0.05, **p < 0.01, ***p < 0.001, ns: no statistically significant difference).

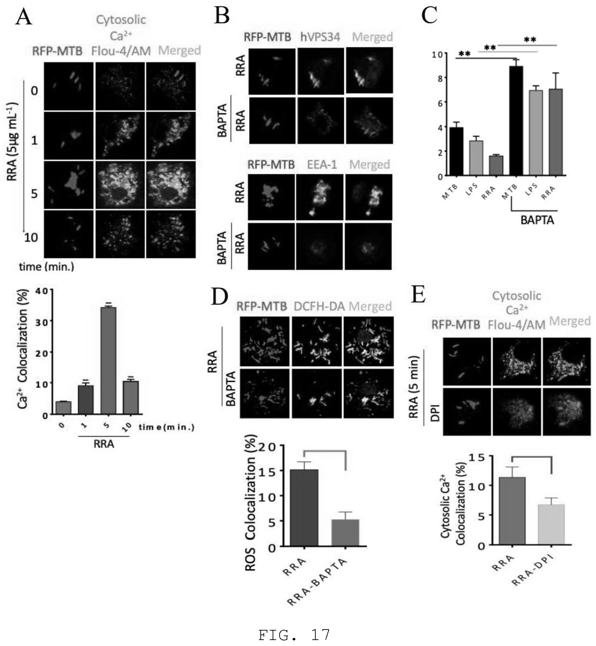

FIG. 17 shows the effect of RRA on the increase in intracellular Ca²⁺ that induces hVPS34 and EEA1 colocalization in MTB-infected BMDMs. (A) BMDMs infected with RFP-MTB [MOI:1] were loaded with Fluo-4/AM and then treated with RRA for the indicated periods, followed by imaging of calcium colocalization using confocal microscopy, and the bar graph indicates the percentage of fluorescence intensity of Fluo-4/AM obtained for infected cells. (B) Infected BMDMs were treated with RRA after application of BAPTA/AM, and then colocalization of hVPS34 or EEA1 was imaged by confocal microscopy. (C) Intracellular *Mycobacterium tuberculosis* growth was measured according to BAPTA/MA pretreatment. (D) Intracellular ROS colocalization induced by RRA according to BAPTA/MA pretreatment in RFP-MTB-infected BMDMs. (E) Intracellular Ca²⁺ colocalization induced by RRA according to DPI pretreatment in RFP-MTB-infected BMDMs. (**p < 0.01).

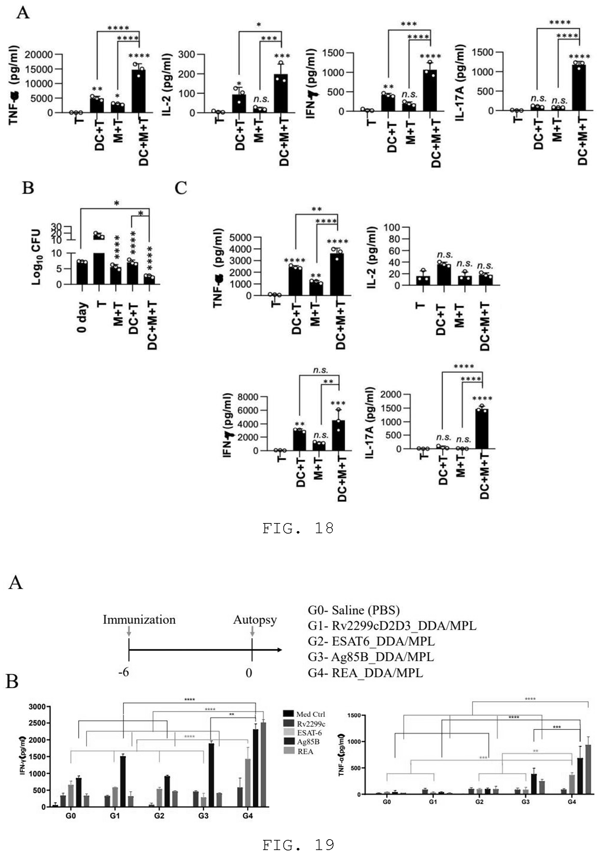

FIG. 18 shows the Th1/Th17 response and antimycobacterial response of T cells activated by REA-treated DCs and macrophages. (A) CD4⁺ splenic T cells isolated from BCG-vaccinated mice (4 weeks after vaccination) were treated for 72 hours at an APC:T cell ratio of 1:10 with REA-treated DCs (1×10⁵/well), REA-treated macrophages (1×10⁵/well), or REA-treated DCs (5×10⁴/well) and macrophages (5×10⁴/well), followed by ELISA measurement of cytokines in the cell lysate supernatants. (B, C) T cells co-cultured for 3 days in the same manner as in (A) were further co-cultured for 72 hours with MTB-infected BMDMs, followed by determination of MTB growth within BMDMs (B) and cytokines in culture supernatants (C). (T: T cell, M: Macrophage, DC: Dendritic cell, mean ± SD (n = 3), ns: no significant difference, *P < 0.05, **p < 0.01, ***p < 0.001, ****P < 0.0001, as compared with T cells).
FIG. 19 shows the in vivo T cell response of antigen-immunized mice. (A) Vaccine administration and necropsy schedule in mice (unit: weeks). (B) Amounts of cytokines in culture supernatants after stimulation of splenocytes isolated from mice at 6 weeks after immunization in (A) with the indicated antigens (2 µg/mL). (**p < 0.01, ***p < 0.001, ****p < 0.0001).

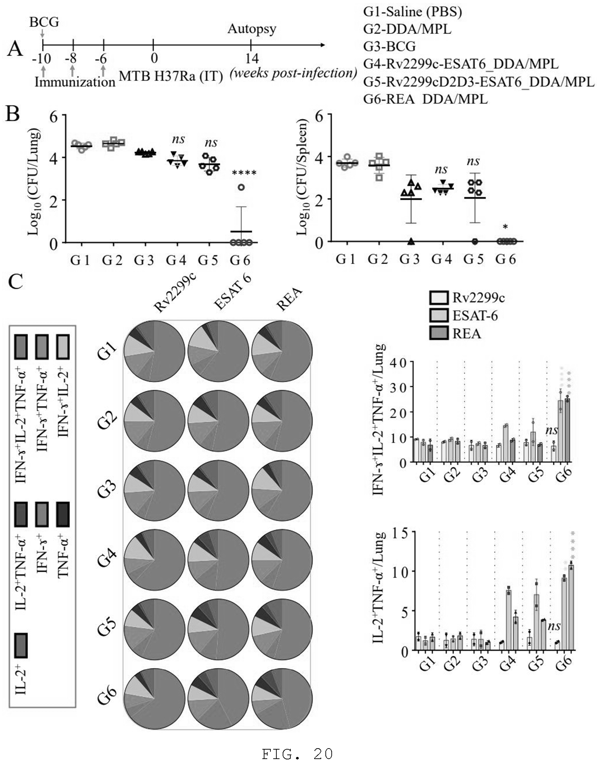

FIG. 20 shows the results comparing the vaccine potential of BCG, Rv2299c-ESAT6, Rv2299cD2D3-ESAT6, and REA (Rv2299cD2De-ESAT6-Ag85B) of the present disclosure. (A) Vaccine administration and necropsy schedule in mice. (B) Bacillary burden in the lungs and spleens of individual mice after initiation of immunization. (C) After stimulating lung cells (2.0×10⁶) with Rv2299c, ESAT-6, and REA (5 µg/mL), respectively, in the presence of GolgiStop, the proportions of antigen-specific CD4⁺ T cells producing IL-2⁺, IL-2⁺TNF-α⁺, IFN-γ⁺, TNF-α⁺, IFN-γ⁺IL-2⁺TNF-α⁺, and IFN-γ⁺IL-2⁺ among cells isolated from the lungs of each mouse group (pie charts), and the proportions of antigen-specific CD4⁺ T cells producing IFN-γ⁺IL-2⁺TNF-α⁺ or IL-2⁺TNF-α⁺ in each group upon stimulation with each antigen (bar graphs). (n = 5). (Mean ± SD, statistical significance determined using an unpaired t-test, *p < 0.05, **p < 0.01, ***p < 0.001, as compared with the MPL-DDA alone group (unpaired t test)).

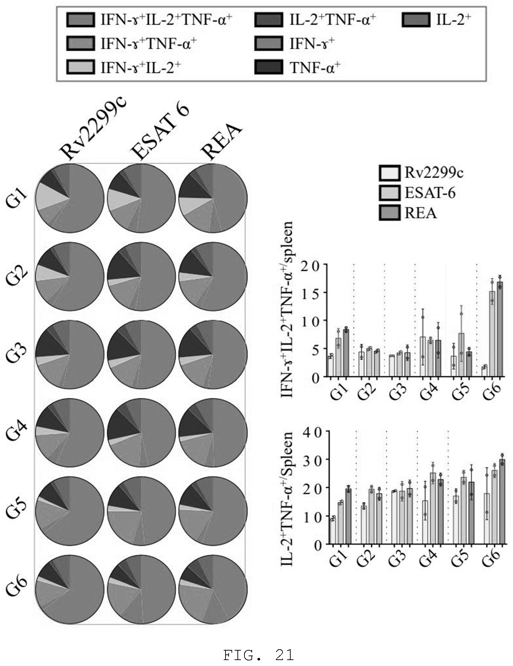

FIG. 21 shows the antigenic immune response of mice immunized with BCG, Rv2299c-ESAT6, Rv2299cD2D3-ESAT6, and REA (Rv2299cD2De-ESAT6-Ag85B). After stimulating splenocytes (2.0×10⁶) with Rv2299c, ESAT-6, and REA (5 µg/mL), respectively, in the presence of GolgiStop, the proportions of antigen-specific CD4⁺ T cells producing IL-2⁺, IL-2⁺TNF-α⁺, IFN-γ⁺, TNF-α⁺, IFN-γ⁺IL-2⁺TNF-α⁺, and IFN-γ⁺IL-2⁺ among cells isolated from the lungs of each mouse group (pie charts), and the proportions of antigen-specific CD4⁺ T cells producing IFN-γ⁺IL-2⁺TNF-α⁺ or IL-2⁺TNF-α⁺ in each group upon stimulation with each antigen (bar graphs). (n = 5). (Mean ± SD, statistical significance determined using an unpaired t-test, *p < 0.05, **p < 0.01, ***p < 0.001, as compared with the MPL-DDA alone group (unpaired t test)).

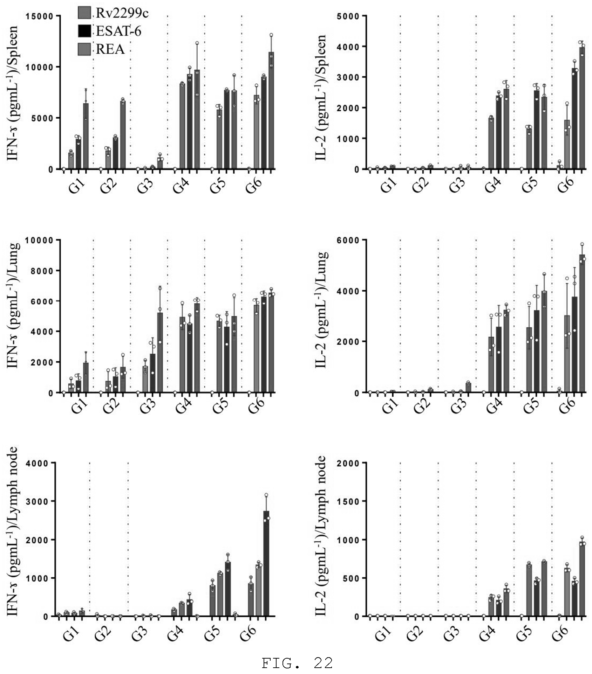

FIG. 22 shows the levels of IFN-γ and IL-2 according to each stimulation in each group after stimulation of cells from lungs, spleens, and lymph nodes with 2 µg of Rv2299cD2D3, ESAT6, or REA at 14 weeks after mouse immunization, as in FIG. 20.

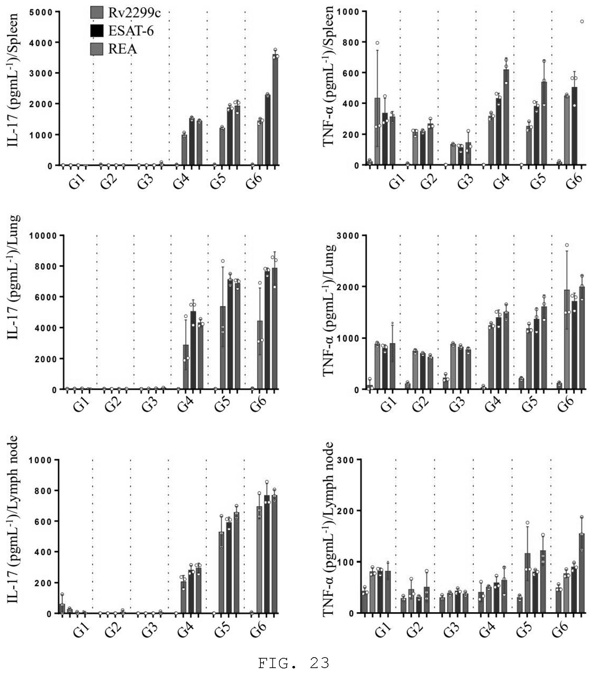

FIG. 23 shows the levels of IL-17 and TNF-α according to each stimulation in each group after the same experiment as in FIG. 22.

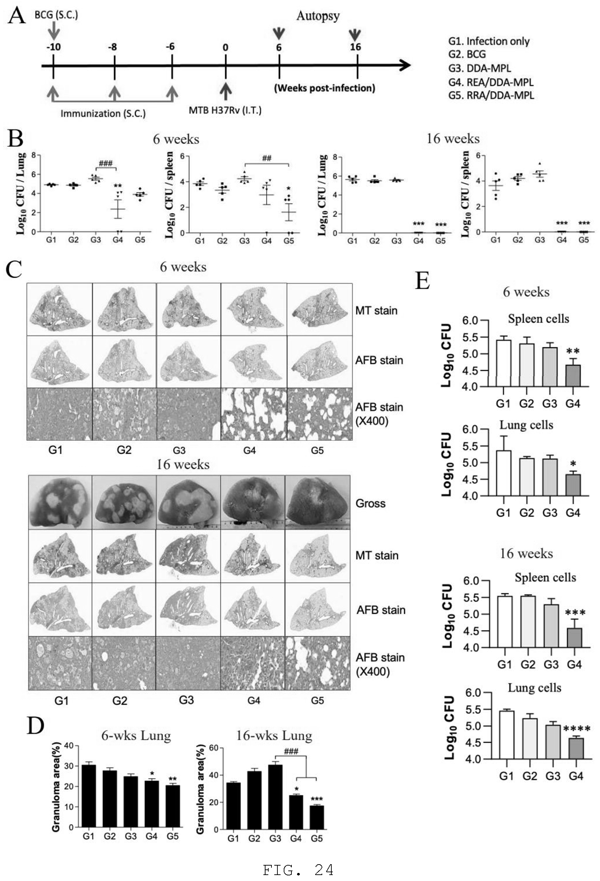

FIG. 24 shows the results of evaluating vaccine efficacy of mice immunized with REA/DDA-MPL and RRA/DDA-MPL against virulent MTB H37Rv infection. (A) Immunization schedule using REA and RRA. (B) Numbers of bacteria in lungs and spleens of each mouse group at 6 weeks or 16 weeks after MTB challenge. (C) Representative histological images of lung lobes of each group using Masson trichrome (MT) and Acid-Fast Bacillus (AFB) staining at 6 weeks or 16 weeks after challenge. (D) Granuloma area (%) in lung sections. (5 mice each). (E) Number of intracellular MTB in BMDMs after co-culturing MTB-infected BMDMs with lung and splenic cells from mice aged 6 weeks or 16 weeks after infection for 3 days. (n = 3, mean ± SD, *p < 0.05, **p < 0.01, ***p < 0.001, ****p < 0.0001, ns: no significant difference).

### Best Mode for Carrying out the Invention

The research team of the present inventors has developed fusion proteins in which a dendritic cellactivating protein or macrophage-activating protein is linked to a T-cell antigen, and has evaluated the efficacy thereof as tuberculosis vaccines. (PMID:34572519, PMID:33105734, PMID:32664238, PMID:32545304, PMID:30862819, PMID:28193909). Recently, as a result of preparing and evaluating an Rv2299c-Ag85B-ESAT6 fusion protein, it was found that the fusion protein induced dendritic cell activation well, enhanced the CD4⁺ T-cell response against Ag85B-ESAT6 by fusing Rv2299c to Ag85B-ESAT6, which is well known as a vaccine composition, and increased the vaccine efficacy of Ag85B-ESAT6 in a short-term vaccine evaluation mouse model (evaluated at 3 weeks after challenge) (PMID:33105734), but there were limitations in maintaining long-term vaccine efficacy, and the molecular weight was large, making purification difficult.

The research team demonstrated that the N-terminal portion of Rv2299c protein, which is a dendritic cellactivating protein, does not exhibit immune activity (PMID:36032688). In addition, as a result of preliminary experiments, it was found that, in constructing a fusion protein, a fusion protein in which the ESAT6 antigen was inserted after Rv2299c showed better vaccine efficacy than a fusion protein in which the ESAT6 antigen was attached to the end of the fusion protein. Accordingly, by modifying the Rv2299c-Ag85B-ESAT6 protein, the N-terminal portion (D1) of Rv2299c was removed to reduce the molecular weight by about 25 kDa, and an Rv2299cD2D3-ESAT6-Ag85B fusion protein was constructed by changing the order of Rv2299cD2D3 and Ag85B-ESAT6 and fusing the same. In addition, in order to determine whether tuberculosis infection is present, the IGRA (IFN-γ release assay) uses ESAT6/CFP-10 antigens that are not present in BCG. Therefore, when ESAT6 is included in a vaccine antigen, even vaccinated individuals have the disadvantage of testing positive in the IGRA. Accordingly, an Rv2299c-Rv3463-Ag85B fusion protein using the Rv3463 antigen instead of the ESAT6 antigen was constructed. The research team has previously reported that the Rv3463 protein may serve as a vaccine candidate antigen as a macrophage-activating protein (PMID: 30862819).

The above two types of fusion proteins, Rv2299cD2D3-ESAT6-Ag85B and Rv2299cD2D3-Rv3463-Ag85B, were designated as REA and RRA, respectively. These proteins were produced and purified in *E. coli*, and immune activity thereof was analyzed using antigen-presenting cells (APCs), and as a result of measuring vaccine efficacy in a mouse model, both fusion proteins exhibited remarkable vaccine efficacy as prime vaccines, in which bacteria were not detected in the lungs and spleen. That is, although the vaccine efficacy of BCG decreases significantly after 14 weeks from the challenge test, the mouse groups vaccinated three times with each of the two vaccines exhibited vaccine efficacy exceeding that of BCG, such that bacteria were eliminated. To date, no tuberculosis vaccine inducing an immune response that completely eliminates bacteria in a mouse model has been reported. Therefore, REA and RRA invented by the research team may serve as tuberculosis vaccines inducing sterilizing immunity, and since both vaccines may replace BCG despite being subunit vaccines, they may be used not only for prevention of adult tuberculosis but also as vaccines for infants and children.

Hereinafter, preferred examples of the present disclosure will be described in detail. However, the present disclosure is not limited to the examples described herein and may be embodied in other forms, and the content introduced herein is provided to fully convey the spirit of the present disclosure.

### <Materials and Methods>

### 1. Cloning for Production of Recombinant Proteins, and Production and Purification thereof

### 1.1 Cloning for Production of Recombinant Proteins by Protein Domain

In order to generate each recombinant protein required for the experiment, the corresponding genes were amplified by PCR using the primers of Table 1 below with genomic DNA from *Mycobacterium tuberculosis* (MTB) H37Rv (ATCC 27294) as a template. The generated PCR products were inserted into the pET-22b (+) vector (Novagen, Madison, WI, USA) using the inserted restriction enzyme sites, and the generated plasmid sequences were analyzed and confirmed.

**[Table 1]**

| Primer | Gene sequence | Restriction enzyme sequence inserted |
|---|---|---|
| Rv2299cD2D3-ESAT6-Ag85B (REA) | | |
| Rv2299c2F | CATATGAACCTGGTCAAGAAATAC | NdeI |
| Rv2299cR | GAATTCG GCAAGGTACGCGCGAGACGTTC | EcoRI |
| ESAT6F | GAATTCGATGACAGAGCAGCAGTGGAAT | EcoRI |
| ESAT6R | AAGCTTTGCGAACATCCCAGTGACGTT | HindIII |
| Ag85BF1 | GATGTTCGCAAAGCTTTTCTCCCGGCCGGGGC | HindIII |
| Ag85BR | TGCTCGAGTGCGGCCGCGCCGGCGCCTAACG | NotI |

| Rv2299cD2D3-Rv3463-Ag85B (RRA) | | |
|---|---|---|
| Rv2299c2F | CATATGAACCTGGTCAAGAAATAC | NdeI |
| Rv2299cR | GAATTCG GCAAGGTACGCGCGAGACGTTC | EcoRI |
| Rv3463F | GAATTCGATGACCAATTGTGCCGCC | EcoRI |
| Rv3463R | AAGCTTAGTCAGTCGGAGCGGCTT | HindIII |
| Ag85BF1 | GATGTTCGCAAAGCTTTTCTCCCGGCCGGGGC | HindIII |
| Ag85BR | TGCTCGAGTGCGGCCGCGCCGGCGCCTAACG | NotI |

### 1.2 Cloning for Production of Fusion Proteins

### (1) pET-22b(+)_Rv2299cD2D3-ESAT6-Ag85B.

Using genomic DNA of *Mycobacterium tuberculosis* (MTB) H37Rv (ATCC 27294) as a template, Rv2299c DNA and ESAT6 DNA were obtained by PCR, and an Rv2299cD2D3-ESAT6 DNA fragment comprising the nucleotide sequence of SEQ ID NO: 1, into which an NdeI restriction enzyme site was inserted at the 5' end and a HindIII restriction enzyme site was inserted at the 3' end, was prepared by an overlapping PCR method and inserted into the pET22b vector. The primers used herein were the same as those shown in Table 1 above, and the amino acid sequence of the polypeptide translated by the nucleotide sequence of SEQ ID NO: 1 is set forth in SEQ ID NO: 2.

### (2) pET-22b(+)_Rv2299cD2D3-Rv3463-Ag85B

Using the Rv2299c, Rv3463, and ESAT6 DNAs obtained by the same method as described above as templates, an Rv2299cD2D3-Rv3463-ESAT6 DNA fragment comprising the nucleotide sequence of SEQ ID NO: 3, into which an NdeI restriction enzyme site was inserted at the 5' end and an XhoI restriction enzyme site was inserted at the 3' end, was finally prepared by PCR and inserted into the pET22b vector. The primers used herein were the same as those shown in Table 1 above, and the amino acid sequence of the polypeptide translated by the nucleotide sequence of SEQ ID NO: 1 is set forth in SEQ ID NO: 4.

### 1.3 Production of Recombinant Proteins

The recombinant plasmids prepared above were transformed into *E. coli* BL21 cells. *E. coli* cells containing the recombinant plasmids were grown in a shaking incubator at 37°C. When a predetermined optical density (OD) at a wavelength of 600 nm was reached, isopropyl-D-thiogalactopyranoside (IPTG; ELPIS-Biotech, Daejeon) was added at a concentration of 1 mM. After 4 to 6 hours, bacterial cells were harvested by centrifugation and suspended in 20 mM Tris-HCl (pH 8.0), 0.5 M NaCl, 5 mM imidazole, 6 M urea, and 1 mM phenylmethylsulfonyl fluoride (Sigma). For purification of a single protein, the same composition was used except for urea. After lysis by sonication, the recombinant proteins were purified by nickelnitrilotriacetic acid (Ni-NTA) agarose chromatography according to the manufacturer's instructions (Qiagen, Chatsworth, CA, USA). Each purified protein was analyzed by sodium dodecyl sulfate polyacrylamide gel electrophoresis (SDS-PAGE), followed by Coomassie brilliant blue staining and Western blot analysis using an anti-His antibody (Santa Cruz). The purified proteins were concentrated and dialyzed against phosphate-buffered saline (PBS, pH 7.4). PBS was used for dialysis of all single proteins. In order to remove endotoxin, the dialyzed proteins were incubated with polymyxin B-agarose (PMB, Sigma) at 4°C for 2 hours. Finally, the purified endotoxin-free recombinant proteins were filter-sterilized and frozen at -70°C. Protein concentration was calculated using a bicinchoninic acid (BCA) protein assay kit (Pierce, Rockford, IL) with bovine serum albumin (BSA) as a standard. Purity of all proteins was evaluated by Coomassie blue (CB) staining and Western blot (WB) using an anti-histidine antibody.

### 2. Culture of Mouse Bone-Marrow-Derived Dendritic Cells (BMDCs)

Bone-marrow-derived dendritic cells (BMDCs) were cultured at 37°C and 5% CO₂ using RPMI 1640 medium (Roswell Park Memorial institute) supplemented with 10% FBS, 1% antibiotics (Welgene), and 0.1% 2-mercaptoethanol, 5 mM HEPES buffer, 1% MEM solution, 20 ng/mL granulocyte-macrophage colony-stimulating factor (GM-CSF), and 2 ng/mL IL-4. Non-adherent cells and loosely adherent proliferating DC aggregates were harvested on day 7 or 8 and used for further experiments.

### 3. Culture of Mouse Bone-Marrow-Derived Macrophages (BMDMs)

BMDMs obtained from femurs and pelvises were cultured in Dulbecco's modified Eagle's medium (DMEM) containing 10% fetal bovine serum (FBS), 50 ng/mL macrophage colony-stimulating factor (M-CSF) (R&D Systems, USA), and 1% antibiotics (Welgene, Republic of Korea) in a cell incubator under conditions of 5% CO₂ and 37°C.

### 4. Preparation of MTB Strains

MTB H37Rv (ATCC 27294) and H37Ra (ATCC 25177) were cultured using 7H9 medium containing 0.5% glycerol, 0.05% Tween-80, and 10% oleic acid, albumin, dextrose, and catalase.

### 5. Preparation of Animals

Specific pathogen-free, 5- to 6-week-old female C57BL/6 mice were housed in the biological hazard animal laboratory of Chungnam National University College of Medicine. Under housing conditions of 12 hours light and 12 hours dark, sterilized standard diet was supplied. The mice were monitored daily, and no mouse exhibited clinical symptoms or disease during the experiment.

### 6. Cell Infection Experiment and Intracellular Mycobacterial Growth Assay

BMDMs were seeded into plates at a density of 1×10⁵ cells per well and cultured, and then infected by treatment with Mtb H37Rv (MOI=1) for 4 hours. Thereafter, in order to remove uninfected *Mycobacterium tuberculosis* remaining outside the BMDMs, the antibiotic amikacin was added to a concentration of 200 µg/mL and treatment was performed for 2 hours, followed by washing with PBS. A specific antigen was added thereto, followed by culture for 3 or 5 days, and the number of intracellular *Mycobacterium tuberculosis* was measured.

Alternatively, BMDCs stimulated by each antigen and splenocytes or T cells isolated from splenocytes were cultured at a ratio of 1:10 for 3 days, and the activated lymphocytes were co-cultured with BMDMs infected with *Mycobacterium tuberculosis* for 3 or 5 days, followed by measurement of the number of intracellular *Mycobacterium tuberculosis*.

For counting intracellular bacteria, BMDMs were collected and treated with distilled water for 30 minutes to lyse the cells, thereby obtaining a lysate. The obtained lysate was serially diluted, spread on 7H10 solid medium, and cultured at 37°C. After culture, the number of colonies formed on the solid medium (colony forming units, CFU) was analyzed.

### 7. In Vitro T-Cell Proliferation Assay

Responder T cells participating in the naïve T-cell response were isolated from total mononuclear cells extracted from BALB/c mice using a MACS column (Miltenyi Biotec). The responder OVA-specific CD4⁺ T cells were obtained from splenocytes of OT-2 mice, respectively. These T cells were stained with 1 µM CFSE (Invitrogen). DCs (2×10⁵ cells/well) treated with OVA peptide in the presence of 10 µg/mL tuberculosis antigen for 24 hours were co-cultured with CFSE-stained CD4⁺ T cells (2×10⁶) at a DC:T cell ratio of 1:10. On day 3 or 4 of coculture, each T-cell batch was stained with PerCP-Cy5.5-conjugated anti-CD4⁺ monoclonal Ab and analyzed by flow cytometry, and the supernatant was harvested and analyzed for levels of IFN-γ, IL-2, and IL-4 by ELISA.

### 8. Enzyme-Linked Immunosorbent Assay (ELISA)

Cytokines produced after stimulation of BMDMs or BMDCs with antigens and cytokines produced under various conditions were detected in culture media for TNF-α, IL-1β, IFN-γ, IL-2, IL-4, and IL-12p70 using a sandwich enzyme-linked immunosorbent assay. Analysis of cytokines in the culture media was performed as recommended by the manufacturers (eBioscience and BD Biosciences). Levels of cytokines released into the culture media were determined by measuring absorbance at a wavelength of 450 nm using a microplate reader. Concentrations of cytokines were calculated using standard curves of recombinant cytokines, and the results were expressed in picograms per milliliter.

### 9. Measurement of Reactive Oxygen Species (ROS)

Intracellular ROS levels were evaluated by staining cells with H2DCFDA (Molecular Probes). BMDM cells were stimulated with the recombinant REA protein and incubated with 10 µM H2DCFDA in PBS for 30 minutes at 37°C in the dark, followed by washing with PBS. Thereafter, the samples were immediately analyzed on a FACS Canto II cytometer, and the data were processed using FlowJo.

### 10. Cell Surface Staining and Flow Cytometric Analysis

Regardless of infection, antigen-treated BMDM or BMDC cells were collected and washed, and then stained with specifically labeled fluorescent-conjugated Abs (monoclonal antibodies for CD80 (16-10A1), CD86 (GL1), MHC class I (34-1-2S), and MHC class II (I-A/I-E, M5/114.15.2), eBioscience). Staining intensity was measured using a flow cytometer (NovoCyte), and the data were analyzed using FlowJo data analysis software (BD Bioscience). In order to confirm cell surface molecules during infection, BMDMs were infected with MTB H37Rv (1×10⁵/well) for 4 hours and then treated with antigen stimulators for 72 hours.

### 11. Immunoblot Analysis

Activated/inhibited/transfected BMDM or BMDC cells were collected and lysed using RIPA lysis buffer containing 50 mM Tris-HCl, pH 7.5, 1 mM EDTA, 150 mM NaCl, 1% Triton X-100, PMSF, 1 mM phenylmethanesulfonyl fluoride, 1% (vol/vol) protease inhibitor cocktail (Thermo Scientific, Rockford, IL, USA), and phosphatase inhibitor cocktail tablets (Sigma-Aldrich, St. Louis). The lysates were centrifuged at 13,475×g for 20 minutes at 4°C. Thereafter, total protein concentration was measured using the Bradford assay (Bio-Rad, Hercules, CA, USA), and equal amounts of protein were separated by SDS-PAGE and transferred onto PVDF membranes (Millipore, Billerica, MA, USA) for immunoblotting. The membranes were blocked for 1 hour at room temperature in Trisbuffered saline containing 0.1% Tween-20 (TBS/T) buffer together with 5% (w/v) nonfat milk, and then probed with primary antibodies for 12 hours at 4°C. Thereafter, the membranes were incubated with corresponding specific peroxidase-conjugated secondary antibodies. The immunoblots were incubated with a chemiluminescent substrate for horseradish peroxidase (HRP) (Millipore), and target proteins were detected using a BioRad ChemiDoc imaging system.

### 12. Immunofluorescence and Confocal Microscopy Analysis

In order to trace maturation of MTB-containing phagosomes, colocalization of RAB5, hVPS34, EEA1, RAB7, and LAMP1 was stained using antibodies against each protein marker. Briefly, BMDMs (2×10⁵/well) were seeded overnight on 18 mm round glass coverslips in 12-well cell culture plates. The cells were then infected with RFP-MTB H37Rv (MOI 1) in antibiotic-free medium and cultured for 4 hours in a 37°C, 5% CO₂ incubator. Thereafter, the cells were gently resuspended in normal medium and treated with LPS, Rv2299c, RRA, or REA for the indicated times. In order to determine colocalization, the cells were fixed with 4% paraformaldehyde in PBS for 10 minutes at room temperature, permeabilized with 0.1% Triton X-100, and blocked with 3% BSA in PBS. Thereafter, rabbit anti-Rab5, anti-hVPS34, anti-EEA1, anti-Rab7, or rat anti-LAMP1 primary antibodies were diluted according to the manufacturer's protocol and applied overnight. Next, the Rab5-, hVPS34-, EEA1-, and Rab7-stained cells were incubated with secondary Alexa Fluor^{®} 488-conjugated antibodies, whereas the LAMP-stained cells were incubated with goat anti-rat Alexa Fluor^{®} 488-conjugated secondary antibodies. Finally, the cell nuclei were stained with 4',6-diamidino-2-phenylindole dihydrochloride (DAPI) and imaged by confocal microscopy. The cells were washed three times with PBS between each step. In order to quantify lysosomes in the cells, the cells were stained with LysoTracker Green (Molecular Probes), and LysoTracker Green uptake was analyzed.

### 13. Mixing of Protein with DDA/MPL

The protein was mixed with dimethyl dioctadecylammonium bromide (DDA) and monophosphoryl lipid A (MPL) according to the method of Andersen et al. (PMID:10639447). After mixing 5 µg of protein, 250 µg of DDA, and 25 µg of MPL, 0.2% triethylamine was added to make a final volume of 200 µL. The mixture was then heated in a 70°C water bath for 30 seconds and then sonicated for 30 seconds. This process was repeated 2 to 3 times. The protein, DDA, and MPL were all mixed immediately before use.

### 14. Vaccine Experiment

In the preventive vaccine experiment, first, the immune vaccine composition to be tested was subcutaneously immunized three times, and after challenge with MTB (H37Ra or H37Rv) 4 or 6 weeks later, the number of bacteria in organs of the mice was measured after a certain period of time. For bacterial infection for the challenge test, the mice were first anesthetized with 1.2% 2,2,2-tribromoethanol (Avertin), and after exposing the trachea through a small midline incision, MTB contained in 50 µL of saline was inoculated intratracheally (IT). For measurement of bacterial counts in the lungs and spleen, the mice were euthanized with CO₂, tissues were excised and homogenized, and the lung homogenates were serially diluted and plated onto Middlebrook 7H10 agar (Difco Laboratories, Detroit, MI) supplemented with 10% OADC (Difco Laboratories), amphotericin B (Sigma-Aldrich, St. Louis), and 2 µg/mL 2-thiophenecarboxylic acid hydrazide (Sigma-Aldrich), followed by incubation at 37°C for 4 weeks, and then colonies were counted. Data for CFU and evaluation of lung inflammation were performed as log₁₀ CFU ± interquartile range (IQR).

### 15. Statistical Analysis

All experiments were repeated at least 3 times. The level of significance for comparison between samples was determined by Tukey's multiple comparison test distribution using statistical software (GraphPad Prism Software, version 4.03; GraphPad Software, San Diego, CA). Data in the graphs are expressed as mean ± SEM, and differences from each value were considered statistically significant at *p < 0.05, **p < 0.01, or ***p < 0.001.

### <Experimental Results>

### 1. Confirmation of Endotoxin Contamination in Purified REA and RRA Fusion Proteins

As described above, *E. coli* was transformed using expression pET plasmids into which DNAs encoding each of the REA and RRA fusion proteins had been cloned, and proteins were purified from the extracts of these *E. coli* cells, followed by SDS-PAGE, and then analyzed by Coomassie blue (CB) staining and Western blot (WB) using an anti-His antibody. The results are shown in FIG. 1A. As shown in FIG. 1A, recombinant protein expression of REA and RRA was confirmed. In addition, in order to confirm whether endotoxin was contaminated during the purification process, BMDMs were pretreated with polymyxin B for 1 hour or left untreated, then cultured with LPS, RRA, or REA for 24 hours, and TNF-α production was analyzed in the culture supernatant by ELISA, and the results are shown in FIG. 1B. As shown in FIG. 1B, it was confirmed that the recombinant proteins of REA and RRA were not contaminated, and at 10 µg/mL, they did not exhibit distinct cytotoxicity to BMDMs.

### 2. Activity of REA and RRA on Antigen-Presenting Cells (APCs)

Since the proteins constituting REA and RRA include proteins activating dendritic cells or macrophages, the activity of each fusion protein on APCs was evaluated. In evaluating activity, single antigenic components constituting the fusion proteins (Rv3463, Ag85B, ESAT6, Rv2299c, or Rv2299cD2D3) and LPS were used as controls.

First, 1 × 10⁵/well of BMDMs or BMDCs were stimulated with 100 ng/mL LPS or 1, 2, 5, or 10 µg/mL of REA and RRA, ESAT-6 (2 µg/mL), Ag85B, Rv2299cD2D3, Rv2299c, or Rv3463 (5 µg/mL each) for 24 hours, and cytokines IL-12p70, TNF-α, and IL-10 in the culture supernatants were measured, and the results are shown in FIGS. 2A and 2C. As shown in FIG. 2A, REA stimulated BMDMs and BMDCs to induce IL-12 and TNF-α production in a concentration-dependent manner, and at a high concentration of REA in BMDCs, IL-10 production was increased. In addition, as shown in FIG. 2C, similarly to REA, RRA also significantly induced production of inflammatory cytokines IL-12 and TNF-α from BMDMs and BMDCs as compared with unstimulated cells, and in the case of IL-10, induced higher production as compared with REA. In particular, in BMDMs, RRA was higher than Rv3463, and in BMDCs, there was no significant difference between RRA and Rv2299c.

The effects of the recombinant proteins REA and RRA on expression of surface molecules MHC class II, CD80, and CD86 in BMDMs and BMDCs were confirmed. Activated BMDMs or BMDCs were stained with anti-CD80, anti-CD86, or anti-MHC class II antibodies, and expression of surface markers was confirmed by fluorescence-activated cell sorting (FACS), and the results are shown in FIGS. 2B and 2D. As shown in FIG. 2B, FACS analysis also showed that expression of surface molecules MHC class II, CD80, and CD86 in BMDMs and BMDCs was significantly increased by REA stimulation. In the case of BMDCs, cytokine production and surface molecule expression produced by the individual antigens Ag85B, ESAT6, and Rv2299cD2D3 were similar to or lower than those of REA. In particular, the expressioninducing ability by Ag85B was the lowest. In addition, as shown in FIG. 2D, surface molecule expression by RRA was significantly increased in both BMDMs and BMDCs as compared with the medium control (MC), and there was no significant difference as compared with the individual antigens Rv3463 or Rv2299c.

Since both REA and RRA comprise Rv2299cD2D3, the activity of dendritic cells matured by each fusion protein was analyzed. Unstimulated DCs (control DCs) and DCs stimulated with Rv2299cD2D3 (5 µg/mL), Ag85B (5 µg/mL), ESAT6 (2 µg/mL), or REA (1 or 2 µg/mL) were co-cultured with naïve T cells for 72 hours (DC:T cell = 1:10), and then production of IFN-γ and IL-10 in the culture supernatants was analyzed by ELISA, and the results are shown in FIG. 3A. As shown in FIG. 3A, T cells activated by BMDCs matured by REA showed significantly higher IFN-γ production and lower IL-10 production as compared with T cells activated by BMDCs matured by other antigens.

In addition, transgenic OVA-specific CD4⁺ T cells isolated from OVA peptide-specific transgenic mice (B6.Cg-Tg(TcraTcrb)425Cbn/J) were stained with CFSE and co-cultured with DCs treated with REA (2 µg) or LPS (100 ng/mL) for 96 hours, followed by pulsing with OVA323-339 (1 µg/mL), and as a result of confirming T-cell proliferation by flow cytometry, as shown in FIG. 3B, it was confirmed that DCs matured with REA effectively and significantly induced proliferation of these T cells. At this time, as a result of measuring concentrations of IFNγ, IL-2, IL-17, and IL-4 in the culture supernatants under each condition by ELISA, as shown in FIG. 3C, production of IL-17, IL-2, and IFN-γ was also significantly high.

In the same manner as for REA above, unstimulated DCs (control DCs) and DCs stimulated with Rv2299cD2D3 (5 µg/mL) or RRA (2 µg/mL) were co-cultured with naïve T cells for 72 hours (DC:T cell = 1:10), and then production of IFNγ, TNF-α, and IL-17 in the culture supernatants was analyzed by ELISA, and the results are shown in FIG. 4A. As shown in FIG. 4A, T cells activated by DCs matured with RRA produced high levels of IFN-γ and TNF-α. In addition, also in experiments using transgenic mice, as shown in FIGS. 4B and 4C, DCs differentiated and matured with RRA well induced T-cell proliferation and Th17 and Th1 responses, and induced production of IL-17, IL-2, and IFNγ.

### 3. Activation of APCs Through TLR2 and TLR4 Pathways

The pathways through which the two proteins, REA and RRA, activate antigen-presenting cells (APCs) were analyzed. It has been reported that various *Mycobacterium tuberculosis* components mediate signal transduction through TLR2 or TLR4 pathways. Accordingly, the signaling pathway through binding of the present fusion proteins to TLRs present on the surface of BMDMs was confirmed.

BMDMs derived from wild-type (WT), TLR2-/-, and TLR4-/- mice were treated with REA (5 µg/mL), LPS (100 ng/mL), or the TLR2 agonist Pam3CSK4 (100 ng/mL) for 24 hours, and then TNF-α, IL-12, and IL-6 production in the culture supernatants was determined by ELISA, and the results are shown in FIG. 5A. As shown in FIG. 5A, LPS, which is a ligand of TLR4, failed to induce cytokine secretion in BMDMs from TLR4-deficient mice, and Pam3, which is a TLR2 ligand, failed to induce cytokine secretion in BMDMs from TLR2-deficient mice. In REA-treated BMDMs lacking expression of both TLR2 and TLR4, cytokine secretion was significantly suppressed as compared with that in WT cells, but was not completely inhibited. This means that at least both TLR4 and TLR2 are simultaneously involved in macrophage activation induced by REA. Next, MAPK activation was confirmed by immunoblotting, and the results are shown in FIGS. 5B to 5E. As shown in FIGS. 5B to 5E, in BMDMs stimulated with REA, p38 and ERK were phosphorylated, and phosphorylation of phosphoinositide 3-kinase (PI3K) and AKT, as well as glycogen synthase kinase-3β (GSK-3β), was also induced. This means that REA ultimately induces activation of macrophages through activation of NF-κB via the PI3K-AKT signaling axis pathway.

In the same manner as described above, BMDMs derived from wild-type (WT), TLR2^{-/-}, and TLR4^{-/-} mice were treated with RRA (5 µg/mL), LPS (100 ng/mL), or the TLR2 agonist Pam3CSK4 (100 ng/mL) for 24 hours, and then TNF-α, IL-12, and IL-6 production in the culture supernatants was determined by ELISA, and the results are shown in FIG. 6A. As shown in FIG. 6A, in the same way as for REA, RRA also significantly suppressed cytokine secretion in BMDMs lacking expression of both TLR2 and TLR4 as compared with WT cells, but did not completely inhibit the same. This means that at least both TLR4 and TLR2 are simultaneously involved in macrophage activation induced by RRA. In addition, as a result of immunoblotting analysis of phospho-p38 (p-p38), p38, phospho-ERK1/2 (p-ERK1/2), ERK1/2, p-PI3K, P-PI3K, p-AKT, p-IκBα, IκBα, and NFκB p65 in membrane fractions in BMDMs and BMDCs, and intracellular localization analysis of the p65 subunit of NF-κB detected by immunofluorescence, as shown in FIGS. 6B to 6D, it was confirmed that RRA also induced phosphorylation of p38, ERK, AKT, and PI3K and ultimately activation of NF-κB in both BMDMs and BMDCs.

Meanwhile, in order to confirm the effects of pharmacological inhibitors on surface marker expression and cytokine production of BMDMs treated with REA and RRA, BMDMs (1×10⁶/well) were treated for 1 hour with each pharmacological inhibitor of p38 (SB203580, 20 µM), ERK1/2 (U0126, 10 µM), and NF-κB (Bay11-7082, 5 µM), or DMSO (vehicle control), and then treated with REA or RRA (5 µg/mL) for 24 hours, followed by determination of levels of co-stimulatory surface markers (CD80, CD86) by flow cytometry and measurement of TNF-α and IL-6 in culture supernatants by ELISA, and the results are shown in FIGS. 7A and 7B. As shown in FIGS. 7A and 7B, although to different extents, inflammatory cytokine production and increased surface molecule expression mediated by RRA and REA were inhibited by MAPK inhibitors [SB203580 (p38 MAPK inhibitor), SP600125 (JNK inhibitor), U0126 (ERK1/2 inhibitor), BAY 11-7082 (NF-κB inhibitor), and LY294002 (PI3K inhibitor)].

### 4. Anti-tuberculosis Activity of REA and RRA

*Mycobacterium tuberculosis* has the ability to survive and proliferate in macrophages, which are phagocytic cells. Accordingly, activation of macrophages to induce killing of phagocytosed bacteria is an important issue in the anti-tuberculosis immune response. Thus, it was confirmed whether intracellular killing of *Mycobacterium tuberculosis* could be induced by stimulating *Mycobacterium tuberculosis*-infected BMDMs with REA or RRA.

BMDMs (1×10⁵ cells/well) were infected with H37Rv at a multiplicity of infection (MOI) of 1 for 4 hours and additionally treated with kanamycin for 2 hours. Thereafter, in order to eradicate extracellular *Mycobacterium tuberculosis*, the cells were washed three times, and then the infected BMDMs were cultured with Rv2299c or REA (5 µg/mL) and LPS (100 ng/mL) for 72 hours, and intracellular MTB growth was examined. As a result, when *Mycobacterium tuberculosis*-infected BMDMs were stimulated with REA for 72 hours, intracellular proliferation of *Mycobacterium tuberculosis* was significantly inhibited as compared with cells stimulated with LPS or Rv2299c (FIG. 8A).

Antigen presentation ability during infection (in BMDMs infected with H37Rv [MOI:1]) was analyzed with respect to expression of surface molecules by two-color flow cytometric analysis. BMDM cells were stained with an F4/80+ antibody, whereas surface molecules were stained with anti-CD80, anti-CD86, anti-MHC class I, or anti-MHC class II antibodies. Each histogram in FIG. 8B is representative of five experiments, and the bar graph shows the percentage of each surface molecule in F4/80+ cells (mean ± SD of five experiments). In addition, at this time, production of cytokines in the culture supernatant was measured by ELISA, and the results are shown in FIG. 8C. As shown in FIGS. 8B and 8C, when *Mycobacterium tuberculosis*-infected BMDMs were stimulated with REA, expression of surface molecules (MHC molecules, CD80, and CD86) and production of inflammatory cytokines and the CCL-2 chemokine were significantly increased as compared with *Mycobacterium tuberculosis*-infected cells. There was no significant difference in these enhancing effects between LPS and REA, but IL-12 was significantly increased by REA stimulation as compared with LPS (FIG. 8C).

In order to confirm the effect of T cells matured by RRA and REA on inhibition of intracellular bacterial growth, BMDMs (1×10⁵ cells/well) were infected with H37Rv at a multiplicity of infection (MOI) of 1 for 4 hours and then additionally treated with kanamycin for 2 hours. Thereafter, in order to eradicate extracellular bacilli, the cells were washed three times, cultured for 72 hours, and then intracellular MTB growth was examined. As a result, as shown in FIG. 9A, also when RRA was treated to *Mycobacterium tuberculosis*-infected BMDMs, intracellular bacterial growth was significantly inhibited as compared with the cell culture control and Rv2299c. At this time, IL-12 production and surface molecule expression were also significantly increased, but there was no distinct difference between Rv2299c and RRA (FIG. 9B). In addition, when T cells matured with RRA or Rv2299cD2D3 constituting RRA were co-cultured with *Mycobacterium tuberculosis*-infected BMDMs, intracellular bacterial growth was significantly inhibited as compared with other conditions (FIG. 9C). The inhibitory effect on bacterial growth was better with T cells differentiated by RRA than with Rv2299cD2D3. At this time, cytokines produced in the culture medium were also significantly increased, but there was no large difference among LPS, Rv2299cD2D3, and RRA.

These results mean that REA and RRA can overcome the inhibitory effects induced by *Mycobacterium tuberculosis* infection, and also induce appropriate activation so that macrophages can eliminate phagocytosed *Mycobacterium tuberculosis*.

### 5. Elucidation of the Anti-tuberculosis Mechanism of REA

### 5.1 REA Induces Early Endosomal Maturation by Recruiting the p38-PI3K Signaling Pathway and Effector Molecules

Maturation of the phagosome begins with recruitment of Rab (Ras-associated protein) molecules to the phagosomal membrane. In order to analyze the maturation, BMDMs infected with *Mycobacterium tuberculosis* expressing red fluorescence (RFP-MTB) were stimulated with LPS, Rv2299c, or REA, stained with a Rab5 antibody conjugated with green fluorescence, and observed by confocal microscopy.

BMDMs were infected with red fluorescent protein-labeled H37Rv strain (RFP-MTB) ([MOI=1]) for 4 hours. Thereafter, the cells on microscope cover glass were treated with LPS (100 ng/mL), Rv2299c (5 µg/mL), or REA (5 µg/mL) for 1 hour. The cells were then stained with Ras-associated protein 5 (Rab5), and endosomal colocalization was imaged by confocal microscopy, and the results are shown in FIG. 10A. The bar graph shows the ratio of Rab5 colocalization. As shown in FIG. 10A, colocalization of *Mycobacterium tuberculosis* and Rab5 was significantly increased when stimulated with REA as compared with other conditions.

As the next step, the location of Vps34 [vacuolar protein sorting 34, type III PI(3)K], which plays an important role in production and accumulation of PI(3)P on the phagosomal membrane, was analyzed. As a result of staining the cells with anti-phosphoinositide 3-kinase vacuolar protein-sorting 34 (anti-hVPS34) under the same settings as above, as shown in FIG. 10B, colocalization of *Mycobacterium tuberculosis* and Vps34 was significantly increased when stimulated with REA as compared with other conditions. Since this process is important for recruitment of EEA1 (early endosomal antigen 1), results of analyzing the location of EEA1 are shown in FIG. 10C. The bar graphs show the ratio of colocalization of hVPS34 or EEA1 with phagosomes containing MTB. As shown in FIG. 10C, REA stimulation increased migration of EEA1 into phagosomes containing *Mycobacterium tuberculosis*.

PI3K and p38 play important roles in the early endosomal maturation process. Accordingly, time-dependent phosphorylation and total protein levels of Class I PI3K and p38 MAPK signaling components were examined by Western blot analysis. BMDMs were treated with or without REA for 0.25, 0.5, 1, 2, and 6 hours and infected with RFP-MTB [MOI=1]. Thereafter, cell lysates were collected and stained with antibodies against p-p38 MAPK, p38 MAPK, p-PI3K, PI3K, and β-actin, and the results are shown in FIG. 10D. As shown in FIG. 10D, when infected only with *Mycobacterium tuberculosis*, p38 was transiently phosphorylated only at the early stage, whereas when stimulated with REA, p38 was activated for a longer period of time, and PI3K showed no distinct phosphorylation upon *Mycobacterium tuberculosis* infection, but was distinctly activated when stimulated with REA.

In addition, BMDMs were pretreated with the SB303580 p38 inhibitor (10 µM) for 1 hour and treated with REA (5 µg/mL) during H37Rv infection. Thereafter, the cells were stained with anti-EEA1, and phagosomes containing MTB were observed by confocal microscopy for colocalization with the indicated marker, and the results are shown in FIG. 10E. As shown in FIG. 10E, when pretreated with the p38-specific inhibitor, REA-induced colocalization of EEA1 and *Mycobacterium tuberculosis* was significantly inhibited. In addition, intracellular bacterial growth was measured in MTB-infected BMDMs pretreated with SB303580 (10 µM) for 1 hour before infection and treated with REA (5 µg/mL) or LPS (100 ng/mL), and the results are shown in FIG. 10F. As shown in FIG. 10F, the inhibitory effect on *Mycobacterium tuberculosis* proliferation induced by REA was also abolished.

### 5.2 REA Induces Acidification of Phagosomes and Fusion with Lysosomes

The next step of endosomal maturation is recruitment of Rab7, which is a late endosome-lysosome-associated small GTPase. When migration of Rab7 is inhibited, fusion of phagosomes with late endosomes is inhibited. BMDMs (1×10⁶/well) were infected with RFP-MTB [MOI:1] for 4 hours and further treated with Rv2299c (5 µg/mL) or REA (5 µg/mL) for 1 hour. The cells were then stained with anti-Ras-associated protein 7 (RAB7), antilysosomal-associated membrane protein 1 (LAMP1), and Lyso Tracker^{™} Green DND-26, and phagosomes containing MTB were observed by confocal microscopy for colocalization with the indicated markers, and the results are shown in FIGS. 11A to 11C. As shown in FIG. 11A, when stimulated with REA, colocalization of Rab7 with phagosomes containing *Mycobacterium tuberculosis* was significantly increased as compared with other conditions. In addition, colocalization of *Mycobacterium tuberculosis* with the lysosomal marker LAMP1 (lysosomalassociated membrane protein 1) was also significantly increased upon REA treatment (FIG. 11B). The acidified environment of late phagosomes fused with lysosomes was analyzed using a pH-sensitive LysoTracker. Since LysoTracker conjugated with a blue fluorophore exhibits weak basicity, it binds to acidified organelles and remains therein continuously. As expected, colocalization of LysoTracker and *Mycobacterium tuberculosis* was significantly increased upon REA treatment as compared with Rv2299c treatment (FIG. 11C).

BMDMs were infected with RFP-MTB [MOI:1] for 4 hours, pretreated with the SB203580 p38 inhibitor or the LY2940029 PI3K inhibitor for 1 hour, then treated with Rv2299c or REA, and colocalization of LAMP1 with *Mycobacterium tuberculosis* is shown in FIGS. 11D and 11E. As shown in FIGS. 11D and 11E, when pretreated with the p38 and PI3K inhibitors, REA-increased colocalization of LAMP1 was significantly inhibited. These results mean that the signal pathway of p38-PI3K is involved in fusion of phagosomes containing *Mycobacterium tuberculosis* with lysosomes.

### 5.3 REA Induces Localization of EEA1 Through Increase of Intracellular Ca²⁺

Intracellular Ca²⁺ acts as various signaling molecules. In particular, it plays an important role in molecules related to fusion between phagosomes and lysosomes and in activation of NADPH oxidase. Accordingly, the Ca²⁺ concentration in *Mycobacterium tuberculosis*-infected BMDMs was measured using Fluo-4/AM (a dynamic single-wavelength fluorescent Ca²⁺ indicator). BMDMs (1×10⁶/well) on 18 mm coverslips were infected with RFP-MTB [MOI:1] for 4 hours, and then loaded with the calcium indicator Fluo-4/AM for 30 minutes. Thereafter, the cells were treated with REA (5 µg/mL) for 0, 1, 5, and 10 minutes, and colocalization of calcium was imaged using confocal microscopy, and the results are shown in FIG. 12A. The bar graph represents the percentage of fluorescence intensity of Fluo-4/AM obtained from infected cells according to REA treatment. As shown in FIG. 12A, when REA was treated, intracellular Ca²⁺ increased, reached a peak at 5 minutes, and then decreased. The same number of BMDMs as described above were treated with BAPTA/AM, a Ca²⁺ chelator, and then treated with REA for 1 hour. Thereafter, the ratio of colocalization of EEA1 was imaged using confocal microscopy, and the results are shown in FIG. 12B. The bar graph represents the percentage of fluorescence intensity of EEA obtained from infected cells according to treatment with the Ca²⁺ chelator. As shown in FIG. 12B, when pretreated with BAPTA, which is a calcium chelator, REA-increased colocalization of EEA1 was inhibited. In addition, intracellular bacterial growth according to BAPTA/MA treatment 1 hour before infection was measured and is shown in FIG. 12C. As shown in FIG. 12C, when pretreated with BAPTA, which is a calcium chelator, the inhibitory effect of REA on intracellular proliferation of *Mycobacterium tuberculosis* also disappeared.

### 5.4 REA Induces ROS Production in Mycobacterium tuberculosis-Infected Cells

Reactive oxygen species (ROS) and nitric oxide (NO) are materials mediating killing of intracellular bacteria. NADPH (nicotinamide adenine dinucleotide phosphate) oxidase (NADPH oxidase, NOX2) is an electron transport chain that delivers electrons into phagosomes in professional phagocytes to produce superoxide. An increase in intracellular Ca²⁺ initiates production of ROS and NO through activation of NADPH oxidase on the phagosomal membrane.

BMDMs (1×10⁶/well) on 18 mm coverslips were infected with RFP-MTB [MOI:1] for 4 hours and then confirmed by dihydrodichlorofluorescein (DCDF, 10 µM) fluorescent staining, and the results are shown in FIG. 13A. As shown in FIG. 13A, although *Mycobacterium tuberculosis* infection itself did not induce ROS production, when REA was treated to *Mycobacterium tuberculosis*-infected cells, ROS in the phagosome increased. In addition, as a result of measuring the NO level in culture supernatants 72 hours after REA or LPS treatment, when REA was treated to *Mycobacterium tuberculosis*-infected cells, the concentration of NO released into the phagosomal cell culture medium also increased (FIG. 13B). When pretreated with DPI (diphenyleneiodonium), which is a NOX enzyme inhibitor, localization of ROS into the phagosome induced by REA was significantly inhibited (FIG. 13C), and the same effect was observed when treated with N-acetyl-L-cysteine (NAC, 10 mM), which is an ROS scavenger (FIG. 13A). In addition, as a result of measuring intracellular bacterial growth regardless of the presence or absence of NAC 1 hour before infection, the inhibitory efficacy of REA on intracellular bacterial proliferation was also partially abolished by NAC (FIG. 13D).

Taken together, the above results suggest that the inhibitory efficacy of REA on intracellular proliferation of *Mycobacterium tuberculosis* is attributable to promotion of fusion and acidification of phagosomes and lysosomes, and that ROS and NO are also involved, and that the p38-PI3K and Ca²⁺ signaling pathways are involved in induction of such responses.

### 6. Elucidation of the Anti-tuberculosis Mechanism of RRA

### 6.1 RRA Induces Phagosome Maturation Through the p38-PI3K Signaling Pathway

Since RRA, similarly to REA, also inhibits intracellular proliferation of *Mycobacterium tuberculosis*, BMDMs infected with *Mycobacterium tuberculosis* (RFP-MTB) were stimulated with Rv2299c or REA, stained with a Rab5 antibody conjugated with green fluorescence, and observed by confocal microscopy.

BMDM cells were infected with red fluorescent protein-labeled MTB H37Rv strain (RFP-MTB) ([MOI=1]) for 4 hours and then treated with RRA (5 µg/mL) or Rv2299c (5 µg/mL), followed by staining with Ras-associated protein 5 (RAB5), and endosomal colocalization was imaged by confocal microscopy, and the results are shown in FIG. 14A. The bar graph shows the ratio of RAB5 colocalization induced by REA as compared with Rv2299c and the untreated control. As shown in FIG. 14A, colocalization of *Mycobacterium tuberculosis* and Rab5 was significantly increased when stimulated with RRA as compared with other conditions.

In addition, in order to analyze the related signaling pathway, time-dependent phosphorylation and total protein levels of Class I PI3K and p38 MAPK signaling components were examined by Western blot analysis. As shown in FIG. 14B, RRA significantly increased phosphorylation of p38 and PI3K in BMDMs, and although infection with *Mycobacterium tuberculosis* itself did not show distinct phosphorylation of PI3K, PI3K activation was also significantly maintained when RRA was treated to *Mycobacterium tuberculosis*-infected BMDMs. In addition, phosphorylation of p38 was also transient when induced by *Mycobacterium tuberculosis* alone, but when RRA was treated, activation was continuously maintained. Furthermore, when pretreated with the p38 inhibitor (SB203580) and the PI3K inhibitor (LY294002), phosphorylation of p38 and PI3K induced by RRA was significantly inhibited (FIG. 14C), and the inhibitory effect of RRA on intracellular bacterial proliferation was abolished by pretreatment with the p38 inhibitor (FIG. 14D).

BMDM cells were infected with red fluorescent protein-labeled MTB H37Rv strain (RFP-MTB) ([MOI=1]) for 4 hours and then treated with RRA (5 µg/mL) or Rv2299c (5 µg/mL) for 1 hour, followed by pretreatment with LY2940029 (20 µM) or SB203580 on microscope cover glass, and staining with anti-hVPS34, anti-EEA1, anti-Rab7, or anti-LAMP1 antibodies and Lyso Tracker^{™} Green DND-26, and endosomal colocalization was analyzed by confocal microscopy, and the results are shown in FIGS. 15A to 15F. As shown in FIGS. 15A and 15B, colocalization between the phagosome containing *Mycobacterium tuberculosis* and class III PI3K hVPS4 and EEA1, which are next effector molecules involved in phagosome maturation, was significantly increased in cells stimulated with RRA as compared with untreated cells or cells stimulated with Rv2299c alone. These colocalizations were significantly inhibited by pretreatment with LY294002, which is a PI3K inhibitor, and the bacterial proliferation inhibitory effect of RRA was also abolished (FIG. 15C). Colocalization between Rab7, which is a late endosomal marker, and the phagosome containing *Mycobacterium tuberculosis* was also significantly increased upon RRA treatment as compared with other conditions (FIG. 15D). Taken together, the above results indicate that RRA induces maturation of phagosomes containing *Mycobacterium tuberculosis* through the p38 and PI3K signaling pathway.

In addition, colocalization of *Mycobacterium tuberculosis* with the lysosomal marker LAMP1 (lysosomalassociated membrane protein 1) was also significantly increased upon RRA treatment (FIG. 15E). As a result of analyzing the acidified environment of late phagosomes fused with lysosomes using pH-sensitive LysoTracker, as shown in FIG. 15E, colocalization of LysoTracker and *Mycobacterium tuberculosis* was significantly increased upon RRA treatment as compared with Rv2299c treatment. Moreover, when pretreated with the p38 inhibitor, RRA-increased colocalization of LAMP1 was significantly inhibited. These results mean that the signaling axis of p38-PI3K is involved in fusion of phagosomes containing *Mycobacterium tuberculosis* with lysosomes.

### 6.2 RRA Induces ROS Production in Mycobacterium tuberculosis-Infected Cells

It was analyzed whether RRA, like REA, also induces ROS production. BMDMs (1×10⁶/well) on 18 mm coverslips infected with RFP-MTB [MOI:1] for 4 hours were pretreated with NAC or DPI (10 µM) and then stimulated with LPS or RRA for 4 hours. Thereafter, intracellular ROS levels were measured by a DCDF (10 µM) fluorescence-based assay, and the results are shown in FIGS. 16A and 16B. As shown in FIG. 16A, as a result of stimulating *Mycobacterium tuberculosis*-infected BMDMs with RRA, significantly higher ROS production was induced as compared with LPS, and when pretreated with DPI, which is a NOX enzyme inhibitor, ROS production was completely inhibited. In addition, when RRA was treated to *Mycobacterium tuberculosis*-infected BMDMs, the increased ROS production was also inhibited by pretreatment with NAC (FIG. 16B). In addition, although nitrite in the cell culture medium was not increased by *Mycobacterium tuberculosis* or RRA alone, when RRA was treated to *Mycobacterium tuberculosis*-infected cells, nitrite production was significantly increased (FIG. 16C). Further, as a result of measuring intracellular bacterial growth in BMDMs treated with RRA or LPS after pretreatment with NAC 1 hour before infection, the inhibitory effect of RRA on intracellular proliferation of *Mycobacterium tuberculosis* was abolished by NAC pretreatment (FIG. 16D). In addition, as shown in FIG. 16E, it was confirmed that increased activation of p38 and PI3K when RRA was treated to BMDMs or *Mycobacterium tuberculosis*-infected BMDMs was inhibited by DPI pretreatment.

### 6.3 Increased Intracellular Ca²⁺ by RRA Induces Localization of hVPS34 and EEA1

It was analyzed whether RRA, like REA, can also induce an increase in intracellular Ca²⁺. The Ca²⁺ concentration in *Mycobacterium tuberculosis*-infected BMDMs was measured using Fluo-4/AM (a dynamic single-wavelength fluorescent Ca²⁺ indicator). BMDMs (1×10⁶/well) on 18 mm coverslips were infected with RFP-MTB [MOI:1] for 4 hours and loaded with the calcium indicator Fluo-4/AM for 30 minutes. Thereafter, RRA (5 µg/mL) was treated for the indicated periods, and colocalization of calcium was imaged using confocal microscopy, and the results are shown in FIG. 17A. The bar graph indicates the percentage of fluorescence intensity of Fluo-4/AM obtained for infected cells. As shown in FIG. 17A, when RRA was treated, intracellular Ca²⁺ increased at 1 minute, reached a peak at 5 minutes, and then decreased. In addition, when infected BMDMs were pretreated with BAPTA/AM, which is a calcium chelator, colocalization of hVPS4, which is class III PI3K, and EEA1 increased by RRA was inhibited (FIG. 17B). Moreover, the inhibitory effect of RRA on intracellular proliferation of *Mycobacterium tuberculosis* was also abolished, and intracellular bacterial proliferation increased when BAPTA was pretreated under both the cell culture medium control and LPS-treated conditions (FIG. 17C).

It is known that Ca²⁺-mediated signaling is involved in the mechanism of ROS production. When BMDMs infected with RFP-MTB were pretreated with BAPTA, colocalization of ROS produced by RRA with phagosomes containing *Mycobacterium tuberculosis* was significantly inhibited (FIG. 17D). In addition, when pretreated with DPI, which is a NOX enzyme inhibitor, colocalization of Ca²⁺ increased by RRA was also significantly inhibited (FIG. 17E). These results indicate that Ca²⁺ signaling is involved in phagosome maturation induced by RRA and is also closely associated with the ROS-mediated signaling pathway.

### 7. Evaluation of Vaccine Efficacy of REA and RRA

The present inventors have previously demonstrated that dendritic cells matured by the Rv2299c protein effectively induced T-cell activity capable of eliminating intracellular *Mycobacterium tuberculosis*, and that the Rv2299c-ESAT6 fusion protein actually exhibited excellent BCG booster vaccine efficacy (PMID:28193909). In addition, it was observed that Rv2299c-Ag85B-ESAT6, in which Rv2299c was fused to the Ag85B-ESAT6 fusion protein, increased the vaccine efficacy of Ag85B-ESAT6 in a short-term vaccine evaluation mouse model (evaluated at 3 weeks after challenge) (PMID:33105734). The present inventors obtained preliminary results showing that, when constituting various fusion proteins, vaccine efficacy was superior when another protein was inserted after Rv2299c-ESAT6 rather than between Rv2299c and ESAT6. Based on these results, the antigen linkage order was changed to construct the Rv2299c-ESAT6-Ag85B fusion protein, and finally, since smaller molecular weight is advantageous for commercialization, REA, which is an Rv2299cD2D3-ESAT6-Ag85B fusion protein in which ESAT6-Ag85B is linked to Rv2299cD2D3 lacking the N-terminal (D1 region) of Rv2299c, was constructed. In addition, RRA, which is an Rv2299cD2D3-Rv3463-Ag85B fusion protein using Rv3463 instead of the ESAT6 antigen used as a stimulating antigen in the IGRA test commonly used in place of the tuberculin skin test, was constructed.

REA and RRA may effectively enhance Th1 and Th17 responses to the epitopes contained in each fusion protein through activation of not only dendritic cells but also macrophages, and this may provide vaccine efficacy as a prime vaccine rather than as a BCG booster vaccine. Since it is known to date that a subunit vaccine cannot replace BCG, which is a prime vaccine, if REA and RRA can serve as prime vaccines capable of replacing BCG as subunit vaccines, they may be developed not only as vaccines for adults but also as vaccines for infants and children.

In the course of the above anti-tuberculosis activity analysis, REA and RRA exhibited similar effects and were capable of inducing simultaneous activation of dendritic cells and macrophages. Accordingly, it was verified whether REA and RRA could also increase anti-tuberculosis activity when they induce simultaneous activation of these cells.

First, BCG-vaccinated mice were sacrificed at 4 weeks after vaccination, spleens were excised, and CD4⁺ T cells were isolated therefrom. The CD4⁺ T cells were treated for 72 hours at an APC:T cell ratio of 1:10 with REA-treated DCs (1×10⁵/well), REA-treated macrophages (1×10⁵/well), or REA-treated DCs (5×10⁴/well) and macrophages (5×10⁴/well), and cytokines in the cell supernatants were measured by ELISA, and the results are shown in FIG. 18A. As shown in FIG. 18A, the concentrations of TNF-α, IFN-γ, IL-2, and IL-17 produced from T cells simultaneously activated by the two types of APCs (macrophages and dendritic cells) were significantly higher than those produced from T cells activated by macrophages or dendritic cells activated by REA. After co-culturing for 3 days in the same manner as described above, the T cells were harvested and co-cultured with MTB-infected BMDMs for 72 hours, followed by measuring bacteria in the BMDMs, and the results are shown in FIG. 18B, and measuring cytokines in the culture supernatants, and the results are shown in FIG. 18C. As shown in FIG. 18B, when these T cells were added to *Mycobacterium tuberculosis*-infected macrophages, the inhibitory effect on intracellular bacterial growth was significantly high. In addition, the amounts of TNF-α, IFN-γ, and IL-17 produced at this time were also significantly increased as compared with the case in which T cells activated by a single APC were added (FIG. 18C).

Next, it was analyzed in vivo whether fusion of Rv2299cD2D3 could increase the ability to induce an immune response to Ag85B and ESAT6. As shown in FIG. 19A, mice were randomly divided into five groups and immunized once with G0: saline, G1: Rv2299cD2D3_DDA/MPL, G2: ESAT6_DDA/MPL, G3: Ag85B_DDA/MPL, and G4: REA_DDA/MPL, respectively, and 6 weeks later, the mice were sacrificed and splenocytes were isolated. DDA/MPL was used as an adjuvant. The isolated splenocytes were restimulated with each immunizing antigen, and the produced cytokines IFN-γ and TNF-α were measured, and the results are shown in FIG. 19B. As shown in FIG. 19B, when immunized with REA, IFN-γ and TNF-α were increased for each stimulated antigen except for the Rv2299c antigen, as compared with the case of immunization with a single antigen, confirming that the T-cell response was increased. These results indicate that REA and RRA, which activate both types of APCs, can effectively induce an anti-tuberculosis response, and that Rv2299cD2D3 can increase the T-cell response to other antigens constituting REA.

Since REA and RRA exhibit similar anti-tuberculosis activities, the vaccine potential was evaluated using REA in a mouse infection model employing the weakly virulent *Mycobacterium tuberculosis* strain H37Ra. Vaccine efficacy of the fusion proteins Rv2299c-ESAT6 and Rv2299cD2D3-ESAT6, which are components constituting the REA fusion protein, was comparatively analyzed with that of REA.

As shown in FIG. 20A, mice were divided into six groups of five mice each and immunized with G1: saline (PBS), G2: adjuvant alone (DDA/MPL), G3: BCG alone, G4: Rv2299c-ESAT6_DDA/MPL, G5: Rv2299cD2D3-ESAT6_DDA/MPL, and G6: REA_DDA/MPL, and 6 weeks after the final immunization, a challenge test was performed by direct intratracheal administration of H37Ra *Mycobacterium tuberculosis* (1×10⁶ CFU/mouse). At 14 weeks after infection, the mice were sacrificed, lungs and spleens were excised, and cells were isolated. Thereafter, lung cells (2.0×10⁶) were stimulated with each antigen (5 µg/mL) for 12 hours at 37°C in the presence of GolgiStop, and bacterial counts and immune analyses were performed. As shown in FIG. 20B, no distinct vaccine efficacy was observed in the saline, adjuvant alone (DDA/MPL), BCG alone, Rv2299c-ESAT6_DDA/MPL, and Rv2299cD2D3-ESAT6_DDA/MPL groups. However, in the lungs of mice immunized with REA, *Mycobacterium tuberculosis* was not detected except in one lung, and bacteria were not detected in all spleens. This means that sterilizing immunity for eliminating infection was induced.

It is known that immunological factors reported to be related to vaccine efficacy are proportional to the number of IFN-γ⁺IL-2⁺TNF-α⁺ CD4⁺ T cells and IL-2⁺TNF-α⁺ CD4⁺ T cells. Accordingly, as a result of analyzing antigen-specific multifunctional T cells, as shown in FIG. 20C, when lung and splenic cells were restimulated with ESAT6 and REA, the number of IFN-γ⁺IL-2⁺TNF-α⁺ CD4⁺ T cells was significantly increased in mice immunized with REA as compared with the other antigen-immunized groups (also shown in FIG. 21). When lung cells were restimulated with antigens, the number of IL-2⁺TNF-α⁺ CD4⁺ T cells was significantly increased in the groups immunized with the fusion proteins (FIG. 20C), but there was no significant difference in the spleen (FIG. 21). Similarly, results of measuring the cytokines IFN-γ, IL-2, TNF-α, and IL-17 produced by restimulating lung, spleen, and lymph node cells with antigens are shown in FIGS. 22 and 23. As shown in FIGS. 22 and 23, when immunized with a fusion antigen containing Rv2299c, the levels were much higher than those in the BCG or infection control group, and in particular, it could be confirmed that mice immunized with BCG or adjuvant alone showed no distinct production of IL-2 and IL-17. Taken together, these results confirmed that, when immunized with REA, bacteria were not detected in the lungs and spleen, memory immune cell responses were well maintained up to 14 weeks after challenge, and in particular, the number of IFN-γ⁺IL-2⁺TNF-α⁺ CD4⁺ T cells was proportional to the vaccine efficacy of REA.

Finally, the protective efficacy of REA and RRA was evaluated in a mouse model using the virulent H37Rv *Mycobacterium tuberculosis* strain. The mice were immunized as shown in FIG. 24A, and 6 weeks after the final immunization, a challenge test was performed with *Mycobacterium tuberculosis*. At 6 weeks and 16 weeks after challenge, the number of *Mycobacterium tuberculosis* in the lungs and spleen was measured, and the results are shown in FIG. 24B. At 6 weeks after challenge, bacterial counts in the lungs and spleen were significantly decreased in mice immunized with REA or RRA as compared with the adjuvant control group. At 16 weeks after challenge, BCG and the adjuvant showed no protective efficacy, but surprisingly, bacteria were not detected in the lungs and spleens of all mice immunized with the two vaccines, confirming that sterilizing immunity inducing 99% or more, i.e., 100%, killing of *Mycobacterium tuberculosis* was exhibited.

Histopathological findings also showed results supporting the above bacterial count measurement results. Lung tissues at 6 weeks or 16 weeks after challenge were stained with MT (Masson trichrome) and AFB (Acid-Fast Bacillus), and the results are shown in FIG. 24C. As shown in FIG. 24C, at 6 weeks and 16 weeks, *Mycobacterium tuberculosis* stained as blue dots was abundantly detected in the control group, whereas in the lungs of mice vaccinated with REA or RRA, fewer *Mycobacterium tuberculosis* was observed at 6 weeks as compared with the control group, and at 16 weeks, bacteria could hardly be observed. Also, in the results of analyzing granulomatous lesion areas in lung tissues using an image program, lesion areas were significantly decreased in mice vaccinated with REA or RRA as compared with the control group (FIG. 22D). Finally, lung and splenic cells obtained at 6 weeks and 16 weeks after challenge were isolated and then added to MTB-infected BMDMs to measure the inhibitory effect on intracellular proliferation of *Mycobacterium tuberculosis*, and the results are shown in FIG. 22E. As shown in FIG. 22E, it was confirmed that only lung and splenic cells from mice immunized with REA were able to significantly inhibit intracellular proliferation of *Mycobacterium tuberculosis*. This result proves that the anti-tuberculosis efficacy in immunized mice was maintained.

In conclusion, to date, no tuberculosis vaccine, including attenuated live vaccines, has been reported to induce sterilizing immunity for eliminating infection in a mouse model. In addition, to date, it has been known that a protein-based vaccine cannot replace BCG. However, it was confirmed that the REA and RRA vaccines, although being subunit vaccines, may be used as preventive vaccines against adult tuberculosis regardless of whether BCG has been administered, by inducing an immune response that eliminates infection to a level at which bacteria are not detected in mouse tissues, and also that they can replace BCG as subunit vaccines.

### Sequence Listing Free Text

Sequence listing electronic file attached

## Claims

1. A tuberculosis vaccine composition comprising an Rv2299cD2D3-ESAT6-Ag85B (REA) fusion protein.

2. The tuberculosis vaccine composition of claim 1, wherein the Rv2299cD2D3-ESAT6-Ag85B (REA) fusion protein is encoded by the nucleotide sequence of SEQ ID NO: 1.

3. The tuberculosis vaccine composition of claim 1, wherein the Rv2299cD2D3-ESAT6-Ag85B (REA) fusion protein comprises the amino acid sequence of SEQ ID NO: 2.

4. A tuberculosis vaccine composition comprising an Rv2299cD2D3-Rv3463-Ag85B (RRA) fusion protein.

5. The tuberculosis vaccine composition of claim 4, wherein the Rv2299cD2D3-Rv3463-Ag85B (RRA) fusion protein is encoded by the nucleotide sequence of SEQ ID NO: 3.

6. The tuberculosis vaccine composition of claim 4, wherein the Rv2299cD2D3-Rv3463-Ag85B (RRA) fusion protein comprises the amino acid sequence of SEQ ID NO: 4.

7. The tuberculosis vaccine composition of any one of claims 1 to 6, wherein the tuberculosis vaccine composition induces sterilization of 99% or more against *Mycobacterium tuberculosis*.

8. A recombinant polynucleotide for sterilizing immunity against tuberculosis, comprising the nucleotide sequence set forth in SEQ ID NO: 1 or 3.

9. A recombinant polypeptide for sterilizing immunity against tuberculosis, comprising the amino acid sequence set forth in SEQ ID NO: 2 or 4.
